# EUROPEAN PATENT SPECIFICATION

(11) **EP 3 874 053 B1**
(45) Date of publication and mention of the grant of the patent: **28.09.2022**
(21) Application number: 19798553.4
(22) Date of filing: 28.10.2019
(51) Int. Cl.: C12P 5/02, C10L 3/08, C12N 1/20

(54) **METHOD TO USE INDUSTRIAL CO2-CONTAINING GAS FOR THE PRODUCTION OF A METHANE ENRICHED GAS COMPOSITION**
VERFAHREN ZUR VERWENDUNG VON CO2-HALTIGEM INDUSTRIEGAS ZUR HERSTELLUNG EINER METHANANGEREICHERTEN GASZUSAMMENSETZUNG
PROCÉDÉ D'UTILISATION DE GAZ INDUSTRIEL CONTENANT DU CO2 POUR LA PRODUCTION D'UNE COMPOSITION DE GAZ ENRICHI EN MÉTHANE

(30) Priority: 29.10.2018 DE 102018126953
(43) Date of publication of application: 08.09.2021
(73) Proprietor: ELECTROCHAEA GMBH, 82152 Planegg (DE)
(72) Inventor: FONTAINE, Doline, 8830 Tjele (DK); HOERL, Manuel, 81541 Munich (DE); PESIC, Aleksandra, 85540 Munich (DE); HAFENBRADL, Doris, 82049 Pullach (DE); TAVARES SILVA, Karen, 80804 Munich (DE); AHRENS, Theresa, 81241 Munich (DE)
(74) Representative: Fleuchaus, Andrea
(86) International application number: PCT/EP2019/079433
(87) International publication number: WO 2020/089181

(56) References cited:
- US-A1- 2010 233 775
- US-A1- 2014 377 830

## Description

The present invention refers to a method for producing biogenic methane using carbon-dioxide-containing emissions and/or waste gas.

The pursuit of efficient energy supplying solutions at a lower cost for the environment is a challenge set forth by climate action programs all over the world, from the European Commission, the US groups of businesses and environmental and governmental associations, Japan in its attempt to replace nuclear energy for electricity supply in the aftermath of the tragic incident of Fukushima, to the seven emerging economies and, thus, an endeavor globally undertaken by member countries to counteract climate changes and eventually stabilize the temperature of the planet. The ultimate goal is to fulfill the increasing need for sustainable and renewable energy while counteracting climate change towards the transition to a low carbon modern economy.

The development of enabling legal and technological measures to effectively reduce the need for fossil fuels will also help member countries to comply with the jointly agreed 2020, 2030 and eventually 2050 energy and climate targets and with the wider Energy Union objectives within the respective frameworks and policies. It is then clear that whichever energy source is discovered, engineered, or selected among existent ones, to effectively address the energy demand, this energy source should also fully address the requirements for lower environmental impact.

Methane has the highest energy density per carbon atom among fossil fuels and its potential for energy conversion is far greater than any other natural gas, obtained directly by combustion in presence of oxygen or using fuel cells to produce electricity. Methane as a fossil fuel derives from the metabolic activity of methanogenic microorganisms, which convert carbon dioxide, among others gases and substrates, in deep sea or deep in the earth's crust: this type of methane from geological seepage and natural activities constitutes a large percentage of natural gas; nevertheless, according to a recent upward revision of global fossil fuel methane emissions a comparably large part of the methane present in the atmosphere is of anthropogenic derivation and is liberated into the atmosphere as a result of agriculture, cattle farming, landfills and waste degradation, as well as of coal mining or petroleum drilling, and general fossil fuel handling, transportation, refinement, and combustion operated by the fossil fuel industry.

Methane is capable of 38 times more heat storage/trapping than carbon dioxide on a molar basis over a span of 20 years (McAnulty *et al.,* 2017).

Owing to its heat storage ability, it greatly contributes to the greenhouse effect. Using methane as a fuel is highly convenient in terms of energy yield and its combustion produces a low carbon footprint (low amount of mostly reusable CO₂), making it the cleanest among the fossil fuels; in particular, in the case of biomethane the amount of emitted carbon dioxide produced by its combustion is sufficient for feeding the above-mentioned metabolic processes in a repeating almost self-sufficient cycle; but while biomethane can be produced at the exploitation site, the methane from natural gas that can be conveniently used as fuel needs to undergo further purification processes, then transported with large expenditure of resources.

As natural gas, therefore, methane constitutes a sustainable and renewable energy source and already today increasingly substitutes coal and other fossil fuels. However, the side quests of a dramatic reduction of the environmental impact and viability of its production, storage and transportation are not yet completed.

One of the main technical disadvantages in the exploitation of methane on a large scale is related to e.g. the high level of contaminants remaining in natural gas methane, and therefore a need for costly purification procedures. Furthermore, a long list of still unsolved issues such as a necessity for adequate storage plants for methane, the related needs for better pressurized containment systems, odour from amassing of large quantities of gas, mostly from sulphur-rich contaminants in the natural gas, the risk of explosions, leakages during transportation and storage, and relative inefficiency to scalability of the state-of-the-art production, all these presently hamper and delay the exploitation of methane.

Nevertheless, methane's potential for energy generation has become increasingly relevant in the global market.

Recent research has therefore focused on the development and improvement of methods for producing methane with methanogens, e.g. archaea, which are capable of producing methane from carbon dioxide and hydrogen very efficiently. Presently, the state of the art describes several attempts to enrich gas compositions with methane produced by employing methanogenic microorganisms.

This type of methane production happens in suitable reactors/cells and may be easily set up all over the world, in any setting poor of infrastructures, requiring raw materials generally present in the atmosphere. Most importantly, it generally yields gas compositions enriched with methane and a lower amount of contaminants, wherein such compositions are expected to require less effort until ready to be fed into energy supplying systems. Moreover, its conversion into energy, producing only carbon dioxide and water, is the cleanest among fossil fuels.

Some attempts have been described e.g. in WO 2014016815 A2 where the production of methane through cultures of anaerobic archaea provided in an aqueous growth substrate and cultured at high temperatures leads to a generation of around 20 Vol.% of methane in a gas composition. Some highly specialized anaerobic methanogens deliver under optimized high pressure and high temperatures even up to 30 Vol.% of methane in such gas composition. This process is characterized by the employment of a pressurized aqueous growth substrate for the methanogens and a highly pressurized fluid containing carbon dioxide as feeding source. Besides the relatively low methane production rate, this process is far from being cost effective, owing to the high energy input required for heating and/or pressurizing the culture of specialized methanogenic archaea. Additionally, the utilization of pressurized container poses a serious impediment to the scalability and large diffusion of the methods.

US 2011/0165667 A1 describes a further use of aqueous suspensions containing one or more, or even mixtures, of anaerobic archaea species for the production of methane. It is to note, that the process herein described is characterized by the transformation of pure H2 gas and pure CO₂ gas sources into methane comprising gas compositions. According to this disclosure, electric power from any fossil fuel or other renewable-energy-fueled plant is converted to produce hydrogen, which subsequently is used to feed a culture of specialized methanogenic archaea to produce methane from CO₂ gas under optimized culture conditions. The disclosure further elaborates that typical methanogenic archaea, which are anaerobic by nature, are silenced and stop producing methane, if the culture conditions change and either air or other gas components are fed to the cultures. Particularly, oxygen is known as inhibiting enzymes involved in both hydrogen uptake and methanogenesis, therefore reducing the net microorganism's methane production, therefore the efficiency of the technology, and necessitating of recovery times, where the activity is not completely inhibited by a high concentration of contaminants. Similar effects are described for the presence of carbon monoxide. Consequently, oxygen and carbon monoxide have been singled out and experimental setups have been described to illustrate and explain the effect of silencing the methane production.

While it could be shown that the methanogenic archaea will survive the exposure to these gas components and eventually restore the methane production, these results illustrate that for fulfilling the high standards of methane production for feeding it into the standard gas distribution grid either very pure sources of feeding gas containing only H2 and CO₂ are needed or new engineering solutions need to be found to enrich the methane content above the critical value of e.g. 96% pure methane in the gas composition in compliance with national and/or local requirements of gas grid operators.

As a further development in this regard, US 2014/0377830 A1 describes the deployment of the previously known process regarding the specialized methanogenic archaea in non-strictly oxygen-free environments and, thus, the attempt to adapt the methanogenic process for allowing to use as feeding gas alternative sources such as CO₂ containing emissions from industrial processes. For this, the strains were adapted to effectively produce methane e.g. at atmospheric pressure, and further the production process was adjusted supporting the strains to tolerate contaminants such as oxygen, hydrogen sulfide and carbon monoxide, at different concentrations. With these suitable cultures US 2014/0377830 could demonstrate that methanogenesis was restorable and even sustained or maintained depending on a high rate of feeding hydrogen gas to the culture. While this teaching actually showed that it is possible to restore and maintain some methane production, unfortunately at the same time the solution provided is leading to even more problems, as reported in the following paragraph.

It has been known before and is also described in US 2014/0377830 that under culture conditions, which allow to maintain or restore a methane production in the presence of gas contaminants, and which are e.g. characterized by higher percentage of hydrogen gas input, the effluent gas will contain substantial amounts of hydrogen as well. Such effluent gas mixtures, may not only be explosive due to their oxygen and hydrogen content, but are also unsuitable for feeding into the gas distribution grid due to their lack of purity.

Upgrading biomethane production to an established scalable and reliable renewable energy source proves to remain a challenge, especially owing to the requirement for a continuous supplying process.

Nevertheless, because of its promising potential, the large-scale utilization of biomethane is under attentive political and economic scrutiny, to render the technology remunerative and cost-effective, and harnessing methane has been identified as the most important near-term goal for biochemical engineering. Therefore, effective solutions to the above disadvantages and improvements of production processes driven by methanogenic archaea are urgently needed.

It is thus an object of the present invention to provide a scalable, reliable and continuous production process for methane enriched gas compositions, which can be conducted while using CO₂ containing emissions and waste gas as main feeding source.

Previous publications, and in particular US 2014/0377830, have already described an attempt to use CO₂ containing gases contaminated with oxygen or carbon monoxide in a methane producing system. In this application, the effects of the interruption of methane production, owing to the presence of oxygen or carbon monoxide in the gas fed to the strictly anaerobic methanogenic archaea, are well described. In the context of anaerobic methanogenic archaea, oxygen and, if present, also carbon monoxide are widely known as inhibitors of the enzymes, which are involved in the hydrogen uptake and hamper methanogenesis in general. In the above-mentioned document it was found that feeding the methanogenic cultures with normal air consequently lead to an interruption of methanogenesis, a displacement/reduction of CO₂ and thus an increase of the pH value. In this regard, if pure hydrogen and carbon dioxide are used as process feeds, the methanation process usually operates at a pH value between pH 7.5 and pH 8.5. This pH value range is not specifically set or regulated. In case of replacement of the CO₂ gas by normal air and thus the displacement/reduction of CO₂ from the culture media, methanogenesis is interrupted. Also, in situations where the pure CO₂ gas is not replaced by air but by industrial waste gas containing only a low percentage of oxygen, the consequences on the pH value are comparable, owing to the interruption of methanogenesis in the culture.

Further, it had been previously reported that typically a supply of the basic nitrogen source ammonia is required for the growth and survival of the methanogenic archaea, and furthermore that sulfides are used and considered necessary to maintain a methane producing culture in stationary phase for the replacement of active microorganisms; nevertheless, the addition of these supplements causes new challenges. For example, ammonia is found to increase buffer capacity of methanogenic microorganisms' cultures, therefore increasing the stability of the metabolic processes causing release of methane. The sensitivity to ammonia is found to be dependent on the composition of the culture of methanogenic microorganisms, so that a large quantity of ammonia may inhibit methanation activity while a low amount of ammonia may inhibit acetoclastic methanogenic activity, i.e. the production of methane by conversion of acetic acid (Procházka *et al.,* 2012).

Under artificial culture conditions and applying a feeding scheme with essentially pure H2 and CO₂ gas, an increase of the pH value up to pH 9.00 seemed to be almost uneventful since no significant effect on the methanation was observed. However, the inventors found that under feeding conditions with gas containing a mixture of gases, be it waste emissions or geothermal gas compositions, particularly the content of hydrogen sulfide and depending thereon an uncontrollable formation of insoluble sulfide salts under the typical pH conditions will firstly lead to a decrease in methane productivity and eventually lead to a not-recoverable collapse of the methanogenic archaea culture. Nevertheless, under such conditions the effluent gas shows an increased H₂S amount, unsuitable for its further utilization as a fuel.

It is a further object of current technical development to stabilize and improve the methane production processes employing such industrial waste gas or geothermal gas compositions while still guaranteeing an effluent methane gas fulfilling the strict requirements for it to be fed to the gas distribution grid.

Within this framework of technical advancements, the present invention provides as explicitly specified in the claims teachings on how to improve methane production processes and particularly the methanogenesis of archaea to convert CO₂ containing waste gas into gas compositions enriched with methane or highly pure methane.

According to the present invention gases are to be understood as CO₂ rich emissions and/or waste gas that are found or produced as a side product during activities performed in industrial processes such as fossil fuel or agricultural industries of either microbial fermentation e.g. in the ethanol production, the combustion of fossil fuels e.g. in coal burning energy plants or e.g. as side product of geothermal power plants or e.g. as a result of any human industrial activity resulting in the emission of gas compositions, otherwise dispersing into the atmosphere, such as cattle farming and other agricultural activities. For clarity the term gas, waste gas or emission, as herein understood, also refers to either raw or modified/treated geothermal gas, emitting from or used in geothermal plants, and it is to be further understood as comprising also raw and/or treated geothermal gases, in which e.g. the H₂S content may be reduced. Furthermore, the term gas, waste gas or emission, as herein understood, also refers to emissions of landfills, emissions of lime and cement plants, emissions from steel producing and processing power plants, emissions of garbage or renewable energy firing plants and emissions of geothermal power plants and biogas power plants. Typically, such emissions are considered waste, which need to be inactivated, recycled or purified, to avoid further CO₂ pollution in the atmosphere; therefore, waste gas is also considered to be a gas for the scopes of the present application.

Such gases depending on their source may comprise very different gas compositions. They have primarily in common that they contain a relatively high amount of CO₂ in comparison to air. They may contain a normal (air-like) partial amount of oxygen and/or nitrogen, however depending on their origin they may also be oxygen free. Additionally, they may contain substantial amounts of at least one of the following, particularly carbon monoxide, hydrogen and hydrogen sulfide, other sulphur compounds (sulfides, disulfides, thiols), siloxanes (organic silicon compounds), halogenated compounds, ammonia, and organochlorines, i.e. pesticides and other synthetic organic compounds with chlorinated aromatic molecules.

While the inhibitory effects of oxygen and carbon monoxide are well described regarding the methanation process, particularly the effects of H2S on said process are detrimental for the usage of waste gas as feeding gas for such methanation processes.

As briefly mentioned above, it is known that dissolved sulfide does inhibit hydrogenotrophic methanogenesis of archaea, i.e. production of methane by conversion of carbon dioxide using hydrogen as reducing agent. Although, this form of methanogenesis seems to be robust towards sulfide inhibition compared to acetoclastic methanogenesis (see above), data from Maillacheruvu *et al.,* 1996, indicate that hydrogenotrophic methanogenesis is still affected by sulfide toxicity.

Furthermore, it was demonstrated that the addition of hydrogen sulfide to methanogenic microorganisms can cause non-competitive inhibition of methanogenesis, either utilizing anaerobic sludge or single organisms (Koster *et al.*, 1986; O'Flaherty *et al.*, 1998).

Additionally, sulfide in the form of hydrogen sulfide proved to be more toxic than dissociated sulfide ions, due to the fact-without being bound by the hypothesis -that H2S is the only form that can cross the cellular membrane of archaea. Previously, it has been shown that typical amounts of sulfide concentrations in geothermal gases, namely amounts in the range of 100 to 150 mg/L cause severe inhibition of methanogenesis (Koster *et al.*, 1986). Performing a systematic analysis of varying sulfide additions to an anaerobic reactor operated with anaerobic sludge from a conventional sewage treatment plant (Vila Leopoldina-SP-Brazil), Paula & Foresti (2009) have shown that the specific substrate utilization increased up to total sulfide concentrations of only 50 mg/L. Substrate utilization remained at this level for a while and then gradually decreased for higher values of sulfide addition. Additionally, it is known that sulfide in high concentrations contributes to alleviating the toxicity of metals by metal-sulfide complex formation (Edgcomb et *al.*, 2004).

In order to at least partially overcome the disadvantages of the state of the art listed in the documents mentioned above, the methanation process has been improved according to the method described in the claims.

According to the present invention, the method for producing methane in a bioreactorfrom gases containing CO₂ comprises at least the steps of: culturing methanogenic microorganism in a continuous process; providing CO₂ containing gases, comprising additionally e.g. H2S and/or O₂; feeding the culture of methanogenic microorganisms with additional H₂ in a stoichiometric ratio of CO₂:H₂ between 1:0.6 to 1:5; controlling and regulating the pH value continuously to be kept at a pH value at a given value of below or at pH 10 by adding suitable amounts of an acid and/or base; and a final step of collecting methane or methane enriched gas composition.

In the understanding of the present invention, a bioreactor stands for biological reactor, and is either a bioreaction vessel, or a bioreaction enclosure, or a bioreaction tank, and/or at least a bioreaction chamber, and/or a cell, or a combination thereof, as also intended in the state of the art, able to withstand variations of e.g. temperature and/or pressure, among others, and/or able to maintain whichever imparted values of e.g. temperature, and/or pressure are assigned or have to be maintained, before, after or during the reaction process, and wherein the intended reactions relevant for carrying out the invention may take place. Such reactions are understood as bioreactions as they pertain to the domain of reactions wherein microorganisms are involved, and herein referring to their normal physiology - such as e.g. metabolic fermentation, or aerobic or anaerobic digestion - and that, as such, require suitable environments, suitable cultures of microorganisms, suitable culture mediums and suitable reactants to occur. A bioreactor in the meaning of the invention, performs reliably within the tolerance values of each variable in order to enable the method as disclosed, and it is expected to allow the listed steps to be carried out reliably over time.

A suitable reactor for culturing methanogenic microorganisms, may be, by means of example only, a shake tank bioreactor, a continuous stirred tank bioreactor, an intermittent stirred tank bioreactor, a hollow fiber membrane bioreactor, a bubble column bioreactor, an internal-loop airlift bioreactor, an external-loop airlift bioreactor, a fluidized bed bioreactor, a packed bed bioreactor, a photo-bioreactor, a trickle bed reactor, a microbial electrolysis cell, and/or combinations thereof.

The operation mode of a bioreactor is classified as batch processes, fed-batch processes and continuous processes. According to the different embodiments of the method herein presented, a reactor may be chosen that most closely addresses the specific dynamics of a culture or the convenience by which methane is hereby extracted. A bubble column reactor, or a variant of it, such as an airlift bioreactor, or a continuously stirred tank reactor, and/or any of the above, may be used to conveniently carry out the method as described and a continuous culture is preferred, wherein near-balanced growth, with little fluctuation of nutrients, metabolites, cell number and biomass are observed.

In the meaning of the present invention, and as already discussed above, gases include product and by-product (e.g. waste) gases of industrial and/or geothermal activities, wherein said activities may result in the emission of individual gases or of mixtures of gases, including e.g. raw geothermal gas, therefore including carbon dioxide, carbon monoxide, oxygen, hydrogen sulfide, nitrogen, argon, helium, acetylene, hydrogen and several others in variable amounts, depending on the industrial process.

The use of available gases for the conversion of low energy density gases into a high energy density gas such as methane provides a double advantage: on one side it reuses gases which may contribute to the greenhouse effect if liberated into the atmosphere, in particular in the case of waste gases resulting as a by-product from industrial activities not directly aiming at producing gases for further use; on the other hand, it results into an energy rich gas of high purity, that can be promptly utilized to power further activities producing as waste almost the same amount of low energy density gases that can be further reutilized as a culture feed or nutrient for the production of more biomethane, with a lower impact on the environment than other longstanding methods of energy supply.

According to the present invention, methanogenic microorganisms are cultured in a bioreactor in orderto produce biomethane. Such methanogenic microorganisms, orautotrophic methanogenic microorganisms may be anaerobic archaea or even recently classified aerobic archaea, either in pure strains, or in consortia with a plurality of, i.e. two or more, strains, or in mixed cultures wherein methanation may be also encouraged by syntrophic exchange across different species.

The activity of methanogenic archaea is usually considered strictly anaerobic, with a loss in productivity (expressed as a reduction of methane yield) and eventually death of a culture when, e.g., oxygen contamination occurs. Operating a culture in strictly anaerobic conditions may cause severe restrictions regarding the needed equipment; nevertheless recent findings have shown that methane yield, and therefore the activity levels of a culture, may also be maintained when the oxygen exposure, or exposure to other gas contaminants with the potential to severely reduce methanation in extreme conditions, is carefully controlled so to be regulated and when counteracting measures are taken to maintain critical parameters at operating levels.

Pursuing methods that include the possibility of gas contamination of the culture without reducing the methane yield is therefore meaningful, in the general field of the exploitation of methanogenic microorganisms. The solid finding of the inventors that aerobic manipulation in a pH controlled/regulated culture doesn't limit the viability of said culture, and therefore its methane yield, has increased the possibilities for culturing various methanogens in presence of gas contaminants, as is the case in the present invention.

Autotrophic methanogenic microorganisms are herein intended as microorganisms which derive nutrition from inorganic reactions with their surrounding environment, e.g. by reducing carbon dioxide, to perform biosynthesis of methane. An example of autotrophic microorganisms is given by hydrogenotrophic microorganisms, which derive their nutrition from utilizing hydrogen; in particular, hydrogenotrophic methanogenic microorganisms are able to convert hydrogen and carbon dioxide into methane as part of their metabolic processes. The role of methanogenic microorganisms in the ecosystem is unique as it helps removing excess carbon dioxide and fermentation products in the final stage of decay of organic matter. In absence of methanogenesis large amounts of carbon bound to compounds from decaying matter would accumulate in anaerobic environments.

The class of methanogenic archaea, from the kingdom of Euryarchaeota (encompassing the methanogens and their phenotypically diverse relatives) comprises essentially unicellular microorganisms able to produce methane from a small set of substrates, including hydrogen and carbon dioxide, through their metabolic activity: such activity mainly consists in reducing carbon dioxide with hydrogen and/or other hydrogen compounds to methane.

Cultures of methanogenic archaea suitable for carrying out the method described in the present invention are available in public collections of microorganisms and/or can be alternatively isolated from a number of environmental sources, as reported in the state of the art. Examples of suitable environmental sources of methanogenic microorganisms include anaerobic soils and sands, bogs, swamps, marshes, estuaries, dense algal mats, both terrestrial and marine mud and sediments, e.g. the subsurface of a tidal-flat sediment, deep ocean and deep well floors, sewage and organic waste sites and treatment facilities, and animal intestinal tracts and feces.

Methanogenic archaea suitable for carrying out the method described in the present invention have been taxonomically described under five different classes (orders), as reported below, and namely *Methanobacteria, Methanococci, Methanomicrobia, Methanonatronarchaeia, and Methanopyri,* each of these classes comprising a number of genera, wherein each genus is divided into families, each family encompassing a large number of known and extensively studied, in the meaning of classified, and unknown, in the meaning of unclassified, species.

In the following, those methanogenic archaea particularly suitable for carrying out the method described in the present invention are listed, organized according to their class; the species belonging to different families are listed in brackets after the class name, and separated by a semicolon; each species is then separated by a comma.

According to the present invention, suitable methanogenic archaea are selected from a list of methanogenic archaea from the class of *Methanobacteria* (such as, e.g., *Methanobacterium aarhusense, Methanobacterium aggregans, Methanobacterium alcaliphilum, Methanobacterium arcticum, Methanobacterium beijingense, Methanobacterium bryantii, Methanobacterium congolense, Methanobacterium curvum, Methanobacterium espanolae, Methanobacterium ferruginis, Methanobacterium flexile, Methanobacterium formicicum, Methanobacterium ivanovii, Methanobacterium kanagiense, Methanobacterium locus, Methanobacterium movens, Methanobacterium movilense, Methanobacterium oryzae, Methanobacterium paludis, Methanobacterium palustre, Methanobacterium petrolearium, Methanobacterium subterraneum, Methanobacterium thermaggregans, Methanobacterium uliginosum, Methanobacterium veterum, Methanobacterium sp.; Methanobrevibacter acididurans, Methanobrevibacter arboriphilus, Methanobrevibacter boviskoreani, Methanobrevibacter curvatus, Methanobrevibacter cuticularis, Methanobrevibacter filiformis, Methanobrevibacter gottschalkii, Methanobrevibacter millerae, Methanobrevibacter olleyae, Methanobrevibacter oralis, Methanobrevibacter ruminantium, Methanobrevibacter smithii, Methanobrevibacter thaueri, Methanobrevibacter woesei, Methanobrevibacter wolinii, Methanobrevibacter sp., Methanosphaera cuniculi, Methanosphaera stadtmanae, Methanothermobacter crinale, Methanothermobacter defluvii, Methanothermobacter marburgensis, Methanothermobacter marburgensis str. Marburg, Methanothermobacter tenebrarum, Methanothermobacter thermautotrophicus, Methanothermobacter thermautotrophicus str. Delta H, Methanothermobacter thermautotrophicus str. Winter, Methanothermobacter thermoflexus, Methanothermobacter thermophilus, Methanothermobacter wolfeii, Methanothermobacter sp., Methanothermus fervidus);* and/or from the class of *Methanococci* (such as, e.g., *Methanocaldococcus bathoardescens, Methanocaldococcus fervens, Methanocaldococcus indicus, Methanocaldococcus infernus, Methanocaldococcus jannaschii, Methanocaldococcus villosus, Methanocaldococcus vulcanius, Methanocaldococcus sp; Methanotorris formicicus, Methanotorris igneus, Methanotorris sp.,; Methanococcus aeolicus, Methanococcus maripaludis, Methanococcus vannielii, Methanococcus voltae, Methanococcus sp.; Methanothermococcus okinawensis, Methanothermococcus thermolithotrophicus, Methanothermococcus sp.*)*;* and/or from the class of *Methanomicrobia* (such as, e.g., *Methanocella arvoryzae, Methanocella conradii, Methanocella paludicola, Methanocella sp.; Methanocalculus alkaliphilus, Methanocalculus chunghsingensis, Methanocalculus halotolerans, Methanocalculus natronophilus, Methanocalculus pumilus, Methanocalculus taiwanensis, Methanocalculus sp.; Methanocorpusculum aggregans, Methanocorpusculum bavaricum, Methanocorpusculum bavaricum, Methanocorpusculum labreanum, Methanocorpusculum labreanum, Methanocorpusculum parvum, Methanocorpusculum sinense, Methanocorpusculum sp.,; Candidatus Methanoculleus thermohydrogenotrophicum, Methanoculleus bourgensis, Methanoculleus chikugoensis, Methanoculleus chikugoensis, Methanoculleus horonobensis, Methanoculleus hydrogenitrophicus, Methanoculleus marisnigri, Methanoculleus palmolei, Methanoculleus receptaculi, Methanoculleus sediminis, Methanoculleus submarinus, Methanoculleus taiwanensis, Methanoculleus thermophilus, Methanoculleus sp.; Methanofollis aquaemaris, Methanofollis ethanolicus, Methanofollis formosanus, Methanofollis liminatans, Methanofollis liminatans, Methanofollis tationis, Methanofollis sp.; Methanogenium boonei, Methanogenium cariaci, Methanogenium frigidum, Methanogenium marinum, Methanogenium organophilum, Methanogenium sp.; Methanolacinia paynteri, Methanolacinia petrolearia; Methanoplanus endosymbiosus, Methanoplanus limicola, Methanoplanus sp.; Methanomicrobium antiquum, Methanomicrobium mobile; Methanolinea mesophila, Methanolinea tarda; Methanoregula boonei, Methanoregula formicica; Methanosphaerula palustris; Methanospirillum hungatei, Methanospirillum lacunae, Methanospirillum psychrodurum, Methanospirillum stamsii, Methanospirillum sp.; Candidatus Methanoperedens nitroreducens, Candidatus Methanoperedens sp., etc; Methanosaeta harundinacea, Methanosaeta pelagica, Methanothrix soehngenii, Methanothrix thermoacetophila, Methanosaeta sp.; Methanimicrococcus blatticola, Methanimicrococcus sp.);* and/or from the class of *Methanonatronarchaeia* (such as, e.g., *Methanonatronarchaeum, Methanonatronarchaeum thermophilum, Candidatus Methanohalarchaeum, Candidatus Methanohalarchaeum thermophilum;);* and/or from the class of *Methanopyri* (such as, e.g., *Methanopyrus kandleri, Methanopyrus sp.*)*,* or any combination thereof.

Each of these distinct genera, families and species includes a number of identified and unclassified different microorganisms, or genetically modified strains and related environmental species, with an ongoing sequencing activity to isolate even more species. Further to the mentioned microorganisms suitable to carry out the present invention, a complete list of such classification is available by the Taxonomy Browser of the National Center for Biotechnological Information (NCBI) website.

Moreover, it is possible to select or modify any of the above-listed naturally occurring species, often by simple culturing conditions and natural selection and adaptation mechanisms. In particular embodiments, the final composition of the culture of the reactor may have been modified so that organisms in a particular growth phase have been privileged with respects to others, or according to the state of the reactor, whether dormant or operating/active.

Several studies exist on the genetic manipulation of particular strains in order to adapt them to particular conditions. In general, the approach of the present invention rather relies on naturally occurring microorganisms.

According to some embodiments of the present invention, *Methanothermobacter,* and further *Methanothermobacter thermoautotrophicus, Methanothermobacter marburgensis* and/or mixtures thereof, and/or derivatives thereof revealed particularly suited to carry out the method of the present invention as described or as demonstrated in the subsequent Examples 1-7.

Further according to some embodiments of the present invention, *Methanothermus fervidus, Methanobrevibacter arboriphilicus, Methanococcus* and *Methanocaldococcus sp.,* and further *Methanocaldococcus bathoardescens, Methanocaldococcus fervens, Methanocaldococcus indicus, Methanocaldococcus infernus, Methanocaldococcus jannaschii, Methanocaldococcus villosus, Methanocaldococcus vulcanius* and/or mixtures thereof, and/or derivatives thereof revealed particularly suited to carry out the method of the present invention as described or as demonstrated in the subsequent examples.

In this regard, it is noted that most interestingly the inventors of the present invention found further that *Methanobacteria,* e.g. *Methanothermus fervidus* used in Example 8 was able to perform stable and unexpected excellent methanation when cultured at a regulated pH around 7.0 or slightly above, respectively (cf. Example 8 and 9, pH 7.2 and 7.4 are shown). This finding is in sharp contrast to the state-of-the-art- knowledge, which taught that *Methanothermus fervidus* prefers "*a slightly acidic pH and equal to 6.5, while no growth could be observed at pH above* 7.0" (cf. Anderson et al. 2010, p. 316, right column, lines 4 - 6 and Stetter et al., 1981).

The original culture may have undergone natural modifications in response to the particular conditions in which the culturing has been carried out. Culture conditions are affected by several parameters, such as temperature, pH, pressure, cell density, volume, humidity, salt content, conductivity, carbon content, nitrogen content, vitamin content, amino acid content, mineral content, or a combination thereof, and according to each of these conditions a specific adaptation process may be undertaken by any number of species within the reactor environment. According to the present invention, the method herein disclosed is concerned with the culturing of methanogenic microorganisms in a continuous process, wherein such continuity is understood as continuity in the production of methane and continuity in the culture, wherein no step of separating inactive terminal biomass from active members of the colony is required. It is instead encouraged that dead biomaterial is kept in the reactor together with the active members across several stages of growth, as it is found advantageous that said biomass or biomaterial provide further substrate for the active culture, intensifying nutrition availability. In the understanding of such continuity of methane production and culture, is also included the understanding that a continuous supply of suitable reactants (e.g. industrial gases, geothermal gas) is given to the culture, allowing it to carry out its methane production task without significant alteration of the measured amount of produced methane (i.e. yield of methane) obtained from any cycle of methanogenic activity across the culture and within the operational phases of the reactor. Ensuring a continuous methane production is a relevant feature of the present invention and an advantageous effect of implementing the steps of the method as described. According to the invention, methane is produced by methanogenic archaea from single strains or in mixed cultures, wherein a mixed culture is either a culture where a plurality of, therefore two or more, strains may also be employed, or a culture where a plurality of additional species interact with methanogenic archaea, or any combination thereof.

According to the method herein disclosed, the gases provided to the culture contain CO₂, and are furthermore characterized by additionally comprising H₂S and/or O₂.

Among the gases, as defined above, rich in carbon dioxide and e.g. hydrogen sulfide, among others, raw geothermal gas is also included, which contains suitable amounts of carbon dioxide and hydrogen sulfide, not excluding such gas compositions, which do also comprise traces or contaminations of oxygen.

Additionally, experiments referring to particular embodiments are reported, wherein treated geothermal gas is used, which contains suitable amounts of carbon dioxide and reduced amounts of hydrogen sulfide, not excluding such gas compositions which do also comprise traces or contaminations of oxygen.

In the scope of the present invention it is therefore also comprised that the gases, and in particular CO₂, provided to the culture of methanogenic microorganisms may contain small amounts of further contaminants, without the necessity of being preliminarily purified, because said contaminants contribute to the efficiency of the method.

The method of the present invention does comprise a step of culturing methanogenic archaea, which is based on typical culture conditions for archaea, which have been previously described and which are known to the practitioner. Such conditions are influenced and controlled - according to the skills of a practitioner by common parameters affecting the culture including temperature, pressure, volume, humidity, salt content, conductivity, carbon content, nitrogen content, vitamin content, amino acid content, mineral content, or any combination thereof. According to the present invention, the step of culturing the methanogenic microorganism in the method to produce methane from gases containing CO₂ in a bioreactor comprises: keeping said methanogenic microorganism in a suitable liquid culture medium providing suitable nutrients such as e.g. a nitrogen source and salts; keeping the culture conditions facultatively anaerobic and/or anaerobic; optionally stirring the culture, wherein the stirring of the culture can be carried out regularly, in intervals, continuously, or keeping the soluble culture at least in a certain slow and constant movement; removing metabolic water from the culture continuously; and keeping the temperatures in a range between 32°C and 90°C or between 32°C and 80°C; preferably 50-70°C or around 62°C.

Furthermore, common culture or growth mediums to be provided to the culture of methanogenic organisms may include one or several common inorganic elements, in their elemental forms or in any suitable non-toxic salts thereof selected from the group of sodium, potassium, magnesium, calcium, iron, nickel, cobalt, manganese, zinc, copper, boron, aluminium , molybdenum, tungsten, selenium, chlorine, sources of sulfur, e.g. hydrogen sulfide or elemental sulfur, phosphorus sources, e.g. phosphate, nitrogen sources, e.g. ammonium, nitrate or nitrogen gas. Typical salts utilized for culturing methanogenic organisms according to the present invention are NaCl, NaHCO₃, Na₂HPO₄, NaH₂PO₄ H₂O, Na2S, NH₄OH, N2, and NaO₃, H2S, KCl, MgCl₂, MgSO₄, CaCl₂, and ferrous sulfate.

It was found by the inventors that the regulation of pH, allowed for methanation across the entire activity cycle of the reactor and enabled a continuous yield of methane even in presence of gas contaminants.

Accordingly, the present invention is characterized by a step of controlling the pH value continuously. In this context, controlling is understood in the general common meaning of keeping under constant monitoring the parameters related to the culture and essentially measuring said parameters or status indicators, using common methodologies and measuring instrumentation known in the art, since it might not be sufficient to keep under constant monitoring and therefore only control the pH of the culture; therefore a further embodiment of the present invention comprises in particular regulating the pH value continuously. In the understanding of the present application, regulating is intended as actively maintaining a given value for a parameter, e.g. the pH of the culture, by using appropriate means to do so.

According to one embodiment of the present invention the methanogenic microorganism culture is continuously controlled and regulated, i.e. stabilized to be kept at a pH value at a given value of below or at pH 10 or alternatively at a given value of below or at pH 9, alternatively below or at pH 8 or at a given value of pH 7 by continuously adding suitable amounts of a suitable acid or base. Without being bound by the hypothesis it is believed that regulating the pH of the culture is of particular importance for the preservation of the continuity of the methanation activity, which determines the efficiency of the claimed method.

According to a further embodiment of the present invention the methanogenic microorganism culture is continuously stabilized and/or regulated to keep the pH value by dosing suitable amounts of e.g. NaOH/HCl or NH₄OH/HCl to the culture.

A "given value" according to the invention may be a defined value with given tolerances, tolerances within the measurements system or tolerances due to the variability within the culture or due to the culture diversity, wherein said value is suitable for enabling methanation.

In the context of the present application, methanation, or methanogenesis or biomethanation, is understood as the production of methane or a methane enriched gas composition as carried out by methanogenic microorganisms, such as those included in a list of methanogenic microorganisms suitable to carry out the present invention as described above.

In particular, the methanation reaction, as previously known and as suitable according to the present invention, consumes H₂ and CO₂ at a stoichiometry of 4:1.

The gases or waste gases provided as nutrients for the culture, according to one embodiment of the present invention, referring to geothermal gas, may contain H₂ and CO₂ in proportions different from 4:1. In this case, the raw or treated geothermal gas may still be used, although the substrate in excess might not be fully consumed by methanogenesis. To achieve an optimal H₂:CO₂ ratio of 4:1 in the process feed, (i) geothermal gas may be mixed with external H2 (electrolytic, etc.) and/or CO₂ (from CO₂ containing gases, etc.), depending on which of the two is missing, or (ii) the H₂:CO₂ ratio may be adjusted to 4:1 by separating excess H₂ or CO₂ by suited separation technologies that are well known in the art.

Interestingly, the method according to the present invention proved to remain effective in terms of persistence of methanation activity when carried out using methanogenic microorganism cultures in the continuous process, which had been fed with feeding gas compositions having quite different and surprisingly distinct stoichiometric ratio of H₂:CO₂ from 0.6:1 to ratios of up to 5:1, in particular 1:1, 2:1, 3:1, 3.5:1, 4.3:1.

Additionally, the inventors could demonstrate that even in embodiments with such substantially different stoichiometric ratios H₂:CO₂ being 0.6:1 the process for enriching methane in a gas composition is still stable and ongoing when applying the continuous pH control and regulation (compare to Example 6).

Accordingly, one of the main advantages of the claimed process optimization, including the pH value control and regulation, is the fact that even in case of shortage of feeding gas or interruption of feeding gas the methanation may be reduced, but is still running, or it may even remain constant and can quickly recover.

A methane enriched gas composition produced using the method as described, is intended as a gas composition mostly composed by methane, and/or wherein methane is the main component, and/or a gas composition whose methane content is of 90% in vol. and more, or up to 96% in vol., and in particular the product of the method as described is a gas composition mostly comprising methane which can be directly fed to piping systems for production of heat and power; therefore the methane enriched gas composition of the present invention has a very low content in contaminants, and unlike natural gas, that needs to undergo purification from contaminants that cannot be directly combusted, the enriched methane composition as disclosed can directly interact with oxygen for efficient heat and power generation, to be fed directly to the national energy grids.

In the meaning of the present application, the methane enriched gas composition produced by the method as disclosed corresponds to the understanding of biomethane.

It is considered general knowledge, but should nevertheless be mentioned again, that appropriate means for controlling and/or regulating the value of a parameter in a microorganism's culture vary according to the nature of the parameter. In the case of pH values, regulation and or stabilization occurs by providing alkaline or acidic solutions in accurate amounts calibrated to reach the given pH value. Examples of suitable solutions include but are not limited to potassium hydroxide, sodium hydroxide, hydrochloric acid and sulfuric acid solutions and/or any alkaline or acidic solution that is used or known to be compatible with biological processes.

Regulating, in the meaning of the present application and for the purpose of enabling and stabilizing a continuous methanation activity across the culture and in time, is therefore achieved continuously or in a continuous manner, so that, at any given time, a parameter of the culture, e.g. the pH of the culture, when measured, is found to have the same value as a given value.

Furthermore, it is an advantageous step of the method according to the present invention to remove, regularly or continually, excess moisture and/or an excess of metabolic or so-called free water from the culture media thereby ensuring the correct dilution and/or dispersion of the nutrients in the media. Metabolic water according to the present invention refers to water or H₂O molecules, which are produced by the methanogenic organisms during metabolic activity and the process of methanogenesis.

While the temperatures may vary according to the presence of selected microorganism species within the culture, each of which better thrive within set ranges of temperatures, for most of the methanogenic microorganisms increased temperatures are not detrimental, and they may even assist in optimizing cellular metabolism and thus metabolic turnover or even methanation. In an industrial process a temperature must be controlled by energetic regulation; in this regard it is to be considered a valuable feature to reduce energy expenditure by enabling temperature control. Consequently, it is of substantial importance to balance the optimized culture temperature and the corresponding hydrogen solubility against the costs for energy input. Interestingly, the method of the present invention was found to be most efficient in a temperature range between 32°C and 90°C or between 32°C and 85°C, or alternatively 50 to 70°C or further alternatively around 62°C at atmospheric pressure.

For other temperature or pressure ranges hydrogen solubility can be used as comparative feature. Accordingly, the present invention also refers to a culturing process at a high pressure, e.g. 16 bar, 20 bar, 35 bar, 40 bar or 60 bar and correspondingly, higher temperatures, which would allow the same hydrogen solubility as at a temperature range between 32°C and 90°C or between 32°C and 85°C, or alternatively 50 to 70°C or further alternatively around 62°C at atmospheric pressure.

Methanogenic microorganisms, in general, may live and grow also in a plurality of other and even extreme temperature ranges up to and well above 100°C, e.g. 140°C; accordingly, the above temperature range is an indication of a preferred range, but it is notto be understood as limiting the scope of the invention.

According to a further embodiment of the present invention the methanogenic microorganism is cultured in presence of additionally added sulfide, preferably but not limited to the addition in the form of Na2S and/or additionally added ammonium as nitrogen source, preferably but not limited to the addition in the form of NH₄OH or ammonium chloride.

Ammonium hydroxide is also known as ammonia water and is a colorless aqueous solution. Accordingly, ammonium hydroxide may be used alone or in combination with other nitrogen compounds or even gas to contribute to providing a viable nitrogen source culture medium in the several stages of growth of the colony or culture.

The step of adding to the culture nutrients, in general, or nitrogen sources such as ammonium hydroxide, in particular, is not to be understood as limiting the present invention but should be considered as helpful to the practitioner. Methanogenic microorganism, in general, may live and grow also in the presence of multiple nitrogen sources.

According to another embodiment of the present invention, the methanogenic microorganism are cultured up to a density of microorganisms in the culture of OD₆₁₀ (optical density at 610 nm) being at least 1 and up to 60, wherein such optical density relates to a dry weight of the microorganisms in the culture of at least 0.25 g/L and up to 20 g/L, and ranging in particular from 0.25 to 15 g/L , from 3 g/L to 10 g/L, from 4 g/L to 7 g/L, from 3 g/L to 7 g/L, or from 3 g/L to 15 g/L.

According to a further embodiment of the present invention, the methanogenic microorganism are cultured up to a density of microorganisms in the culture of OD₆₁₀ (optical density at 610 nm) being at least 14 and up to 60, wherein such optical density relates to a dry weight of the microorganisms in the culture of at least 2.5 g/L and up to 20 g/L, and ranging in particular from 2.5 g/L to 15g/L,from 3 g/L to 10g/L,from 4g/L to 7g/L, from 3g/L to 7 g/L, orfrom 3 g/L to 15 g/L.

The optical density of microorganisms in a culture is a viable parameter to measure the cell count or concentration at each time point. A straightforward relationship between a given cell count and the efficiency of the microorganisms in a culture does not appear to have been universally established, nevertheless in the understanding of the results of the method according to the present invention, a high density culture produces advantageous results in terms of methane production and yield.

In particular the optical density (OD) of the culture according to the present invention is measured utilizing common methods and standards known in the art. Optical density, or, rather, turbidity measurements as a form of cell counting are performed using a spectrophotometer, is typically operated around or at 600 nm, but accordingly other wavelengths may be suitable.

Because the optical density may vary according to the measurement setup, it is often useful to indicate the dry weight or biomass density of the microorganisms in the culture as a measure of the amount of cells present in a culture at a given time point or growth phase. It is possible to establish a correlation between measurements of OD of a given culture at a given growth stage and dry weight by building a curve of a number of different OD values of the culture obtained at different concentrations and measuring the dry weight of the dried sample of culture accordingly, using standard methods known in the art. This will provide a set of data points of dry weight as a function of the optical density; the slope of the regression line of such data set usually defines the correlation between dry weight and optical density. According to the inventors, in the present application a value of OD₆₁₀=4 translates, roughly, into a biomass density of 1 g/L.

A high count of microorganisms in the culture determining high optical density may be obtained by keeping the members of the culture in the reactor across the entire stages of their lives, from the several growth stages (active growth phase, stationary growth phase, nearly stationary growth phase) to their terminal stage, so that the remains of the inactive cellular bodies may provide nutrients to the active members of the culture. According to the invention the culture of the methanogenic microorganisms can be guided or led into a density culture with an OD₆₁₀ of at least 1. Alternatively the culture of the methanogenic microorganisms can be guided or led into a high density culture with an OD₆₁₀ of at least 14, but preferably above 20, further also above 30, further above 40 and even up to 60 by adding sufficient nutrient to the culture and simultaneously removing free or metabolic water from the culture. The method of the present invention can thus be suitably performed in culture of one or more strains of methanogenic microorganism, having throughout the various developmental stages a measurable OD₆₁₀ between 1 - 60; further an OD₆₁₀ between 14 - 60; further an OD₆₁₀ between 20 - 60; further an OD₆₁₀ between 30 - 60; further an OD₆₁₀ between 40 - 60; further an OD₆₁₀ between 50 - 60; further an OD₆₁₀ between 1 - 60; further an OD₆₁₀ between 14 - 50; further an OD₆₁₀ between 20 - 50; further an OD₆₁₀ between 30 - 50; further an OD₆₁₀ between 40 - 50; further an OD₆₁₀ between 20 - 40; further an OD₆₁₀ between 30 - 40; further an OD₆₁₀ between 20 - 30; further an OD₆₁₀ between 14 - 20; further an OD₆₁₀ between 1 - 20.

The inventors of the method as described in the present application did show that according to further embodiments of the present invention, the method as described worked particularly well using the methanogenic microorganisms selected from the kingdom of Archaea or archaebacteria, wherein this group comprises *Methanobacterium, Methanobrevibacter, Methanothermobacter, Methanococcus, Methanosarcina, Methanopyrus, Methanothermus* or mixtures thereof, and further the inventors showed that particularly *Methanothermobacter sp., Methanothermus sp. or Methanobrevibacter sp.* proved highly effective.

According to a further embodiment of the present invention the selected methanogenic microorganism is anaerobic, but oxygen tolerant e.g. by adaptation.

As explained above, the methanogenic archaea utilized in the present invention may include both aerobic and strictly anaerobic species, which preserve their ability for methanation in presence of contaminants, and that, when the steps of controlling and regulating the pH of the culture are performed, bypass silencing steps seen in other methods or cultures and maintain the methanation at the expected levels. In the context of the present application, as well as in the general context of the field, silencing is intended as the interruption of methanation activity by methanogenic microorganisms as a reaction to a hostile environment.

According to the present invention, the suitable liquid culture medium of step i. is a moderately saline environment wherein the anion concentration, i.e. the Cl⁻concentration ranges from 12 mmol/L to 300 mmol/L. Alternatively, the suitable liquid culture medium of step i. is a moderately saline environment wherein the concentration of NaCl is in the range of 0.4 g/L to 12 g/L, preferably in the range from 3 g/L to 6 g/L and more preferably around 5.6 g/L.

The chloride anion can be present in the saline solution as the anion of NaCl, MgCl, KCl, NH₄Cl or any other suitable chloride salt known to the skilled person. Particularly, the chloride anion concentration in the moderately saline environment according to the invention compares to the anion concentration provided by a NaCl concentration is in the range from 0.05 g/L, or lower, to 7 g/L, preferably in the range from 3 g/L to 6 g/L and more preferably around 5.6 g/L.

In the understanding of the present application, one further factor for the growth and activity of the microorganisms may also be the amount of salts normally present in the liquid culture medium which allows for reproductivity and continuous increase of biomass, metabolic activities to be performed continuously, and therefore with a desirable conversion efficiency of the reduction of carbon dioxide into methane and a continuously high methane output.

In particular, while most methanogenic microorganism are naturally found in the sea depths, where the salinity is exceedingly high for any other organisms which live at the surface, it was surprisingly found by the inventors that the methanogenic microorganism according to the invention, which are capable of living and thriving in moderately saline environment are particularly suitable in the claimed method.

The use of such methanogenic microorganism according to the invention, which are capable of living and thriving in moderately saline environment is particularly advantageous using modern bioreactor technology; consequences such as e.g. serious corrosion, oxidation and tarnishing damage, associated with the highly saline environments that would be needed for not adapted halophile methanogenic species and that reveal very detrimental to complex bioreactor facilities are avoided.

In the present invention the methanogenic microorganism may be selected from the group of naturally selected or natively adapted microorganisms, an adapted halophilic microorganism, a genetically engineered microorganism, all capable of living and thriving in such moderately saline environment, wherein the NaCl concentration, or salinity, is then lower than or comparable to about half of the NaCl concentration in sea water, and is typically chosen to be about 12 - 14 g/L. In the understanding of the present invention, an adapted microorganism is intended as a microorganism which did not possess, in its initial state, i.e. when being added to the culture, all the same attributes and/or abilities, or any extent thereof, it comes to possess after a period of permanence in the culture. In the same understanding, such attributes and/or abilities might not be a character commonly found in the species of the original microorganism, and nevertheless are acquired after a period or permanence in the culture, said period being variable, depending e.g. on the microorganism and/or the culture, wherein said attributes and/or abilities or any extent thereof vary upon interaction with the population of other microorganisms present in the culture and/or in response to any other parameter and/or element of the culture environment, e.g. the concentration and/or availability of a particular nutrient and/or of a particular contaminant and/or upon interaction with any other similar source of metabolic or any other alteration of the original microorganism, including those as known to the person skilled in the art.

CO₂ containing gas preferably CO₂ containing emission is the carbon source for the methane production and derives preferably from - but not limited to - natural geothermal gas or treated geothermal gas, landfill gas, emissions of coal or fossil-energy-firing plants, emissions of lime and cement plants, emissions from steel producing and processing power plants, emissions of garbage or renewable energy firing plants and emissions of geothermal power plants, biogas plants and fermentation facilities (e.g., breweries, beverage producers and processors).

Typically, the CO₂ content in the gas comprises at least 20% CO₂.

Additionally, the CO₂ containing gas may comprise up to 50.000 ppm H2S, typically the H2S content in the gas used as feeding gas should be between 200ppm and 2000ppm, alternatively between 200ppm and 10000ppm; alternatively between 300ppm and 30000ppm; alternatively between 400ppm and 40000ppm; alternatively between 500ppm and 5000ppm ; alternatively between 200ppm and 20000ppm ; alternatively between 500ppm and 20000ppm or even between 500ppm and 25000ppm and also up to 40.000 ppm H2S.

Additionally, according to further embodiments of the present invention the CO₂ containing gas may comprise up to 5000 mg/L H2S and the H₂S content in the gas used as feeding gas should be between 1 and 100 mg/L H2S, alternatively between 10 and 250 mg/L H2S; alternatively between 150 and 750 mg/L H2S; alternatively between 100 and 1000 mg/L H2S; alternatively between 125 and 850 mg/L H2S; alternatively between 50 and 500 mg/L H2S; alternatively between 125 and 500 mg/L H2S ; alternatively between 125 and 650 mg/L H2S; alternatively between 100 and 2500 mg/L; alternatively between 500 and 1500 mg/L; alternatively between 250 and 3500 mg/L; alternatively between 750 and 3500 mg/L; alternatively between 500 and 4000 mg/L; alternatively between 550 and 4500 mg/L; alternatively between 1000 and 4500 mg/L; and also up to 5000 mg/L H2S.

The CO₂ containing gas may comprise also traces of oxygen. Typically, the CO₂ containing gas may comprise 1%, 2%, 3%, 4% or even 5% of oxygen, however not more than 5% O₂.

In particular, and according to one embodiment the treated geothermal gas according to the present invention is obtained following the so-called Sulfix treating process of raw geothermal gas, carried out at the Hellisheiði geothermal power plant, and contains H2S at concentrations of up to 30000 ppm and oxygen (O₂) at concentrations of up to approx. 1% to 5%, alternatively 2%-4%, alternatively 1%-4%, alternatively 2%-4%, alternatively 2%-3%, or alternatively 1%-3%.

A further typical composition of treated geothermal gas is given in Table 1, included in Example 6. While significantly higher levels of H2S are known to potentially be able to severely inhibit methanogenesis, such already high concentration, together with the presence of oxygen in the given amount, have instead surprisingly proven advantageous when combined with pH regulation, in enabling a continuously occurring methanogenesis activity from the archaea cultured according to the invention.

According to a further embodiment of the present invention either the entire procedure or at least one step may be carried out under atmospheric pressure conditions and/or under pressurized conditions. If according to some embodiments one or more steps of the method according to the invention are carried out in a pressurized atmosphere, then the pressure is chosen to be preferably up to 16 bar, alternatively up to 20 bar, alternatively up to 50 bar, alternatively up to 68 bar, alternatively up to 110 bar or even up to 420 bar.

The collected methane or the methane enriched gas composition produced is essentially free of solid contaminants, small solid particles, like dust and dirt particulates, in suspension, greases, or gaseous contaminants, such as e.g. water vapor, hydrogen sulfide, siloxanes, ammonia and halogen compounds (chloride, fluoride), volatile organic compounds (VOCs), such as e.g. limonene and other terpenes, and other trace contaminants. Such trace components may build up in the plants and piping systems, and may cause corrosion, deposits and damage to equipment, and should therefore be removed upon collection of the effluent gas.

To achieve high purity methane collection, several methods are used, such as e.g. filtration, cryogenic separation, dehumidification, biological oxidation, chemical adsorption, physical adsorption.

The high purity of the biomethane produced according to the invention determines its immediate availability as a source of energy in a continuous production cycle, and thus demonstrates the superiority of the presently claimed method.

The continuous production of high purity methane being the result of the method as herein described, wherein an hydrogen sulfide rich substrate, which optionally may even comprise oxygen is utilized for culturing a species of methanogenic archaea or a mixture of species, to obtain methane virtually free of contaminants or a methane enriched gas composition, immediately ready to reenter the energy cycle, providing a clean fuel with the highest energy yield per carbon atom even in plants and facilities where energy demand is high, and other sources, even those with lower energy yield per carbon atom and high environmental impact, are scarce, costly, impracticable.

The method further may reutilize the products of combustion of methane to provide further substrate to the bioculture, thus restarting the methane production cycle.

Due to these promising results of the method as described above the inventors further studied the effects of culturing said methanogenic microorganisms at different given pH values on the methanization leading to another aspect of the present invention. Thus, in another aspect the present invention provides a method to produce methane from CO₂ or CO₂ containing gases in a bioreactor comprising:
a. culturing a methanogenic microorganism with CO₂ and H₂ at a first given pH value in a range from pH 5.5 to 7.0 to induce rapid replication of the methanogenic microorganism, followed by
b. continuous culturing said microorganism at a second given pH value in a range from pH 7.1 to 10 to increase methane production compared to the methane production of step a;
c. controlling and regulating the pH value to be at the given value;
d. collecting methane or a methane enriched gas composition.

The first pH value ranges from pH 5.5 to 7.0 to induce rapid replication and grow of the methanogenic microorganism and wherein the second pH value ranges from pH 7.1 to 10 to increase and optimize methane production compared to the methane production of step a, i.e. the lower pH range.

It is also described that the second pH value may range from pH 4.5 to 6.5 and optionally is lower than the first pH.

The inventors of the present invention could demonstrate that the shift of the initial first pH range, which is an acidic to neutral pH range, and a second pH range being at an alkaline to neutral pH range, respectively, was substantial for activating the methane metabolism and, thus, caused a pronounced increase in the methane production as compared to the methane production found at the first pH range used during cultivation.

With the method according to the present invention it was thus possible to substantially increase the methane production. It was found that it seems to be the shift regarding the pH value, that during the stages of cultivating the methanogenic organism substantially induces the methane metabolism and thus, leads at the second pH range to an increase of at least 15% to up to 100 %, or at least 30% to up to 80 % or at least 50% to up to 70 % of the average methanation rate compared with the rate at the first pH range.

Interestingly, it was further found that even with a shift of the first pH range being neutral to a second pH range being acidic such comparable prominent increase of the methanation rate could be initiated.

According to said further embodiment of the invention the methanation method comprising said shift of the initial first pH range, being in an acidic to neutral pH range, to a second pH range being at an alkaline to neutral pH range, respectively, was substantially for activating the methane metabolism and thus, caused a pronounced increase in the methane production as compared to the methane production found at the first pH used for cultivation.

### Description of the figures

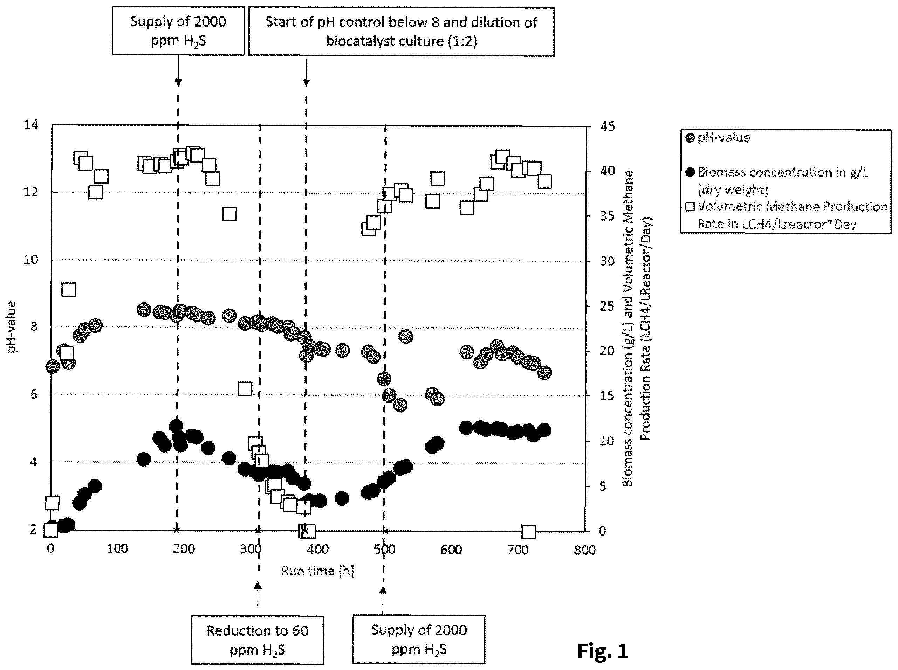

- **Fig.1:**: Methanation reestablishmentfollowing pH regulation in cultures of *Methanothermobacter thermautotrophicus,* after the addition of H2S, according to examples 1 and 2. It can be observed that the collapse of the culture (400 h) could not be avoided by solely reducing the amount of H2S (300 h). The pH regulation plays therefore a relevant role in the reestablishment of methanation and increase of biomass concentration.

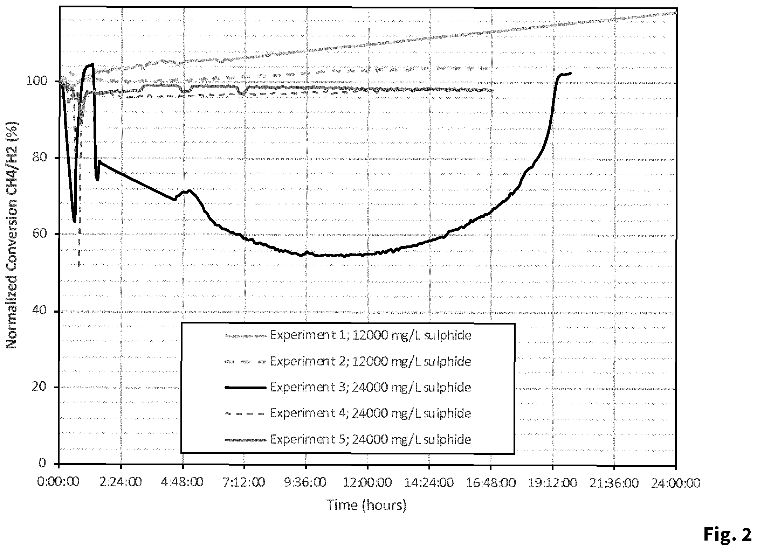

- **Fig. 2:**: Consequences for methanation following addition of up to 24000 mg/L of S²⁻ using cultures of *Methanothermobacter thermautotrophicus.* The data show the surprising performance in terms of metabolic activity of the culture after the addition of very large quantity of contaminants (Na2S), owing to the implementation of pH regulation.

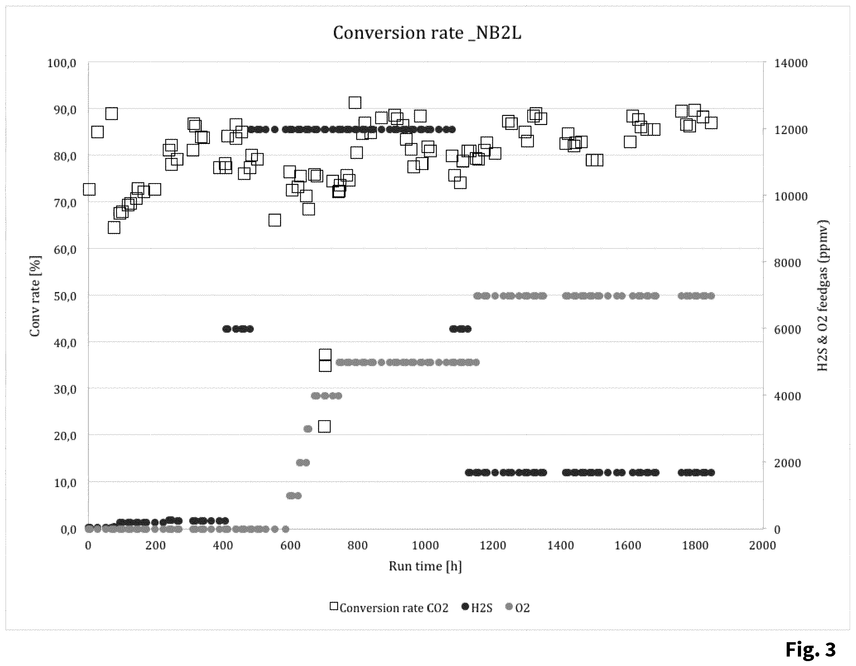

- **Fig. 3:**: CO₂ conversion rate and hydrogen sulfide content and oxygen content present in the process feed gas using cultures of *Methanothermobacter thermautotrophicus.*

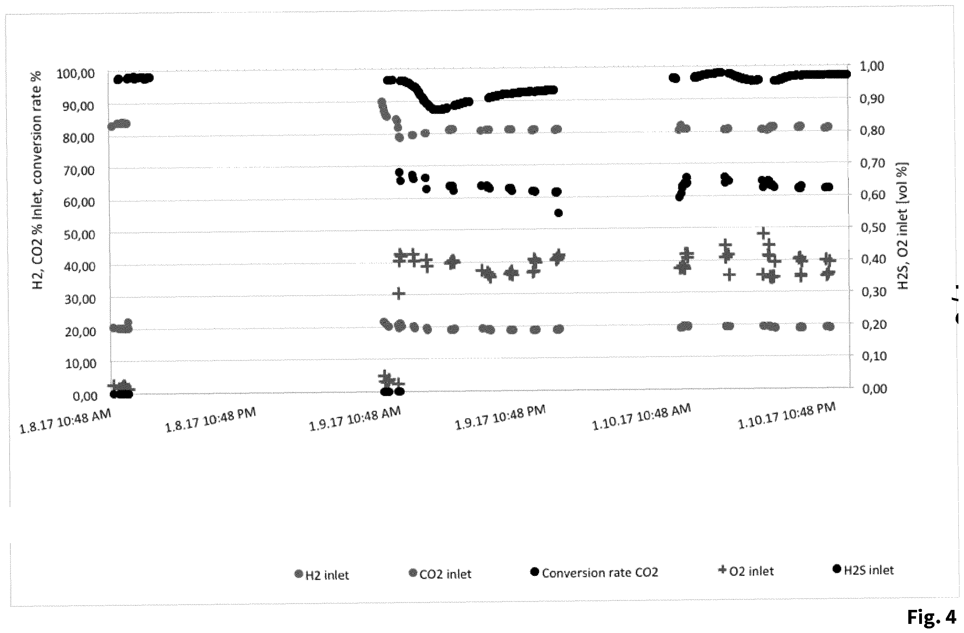

- **Fig. 4:**: Stability of methanation upon switching feeding gas from CO₂ to Sulfix II, treated geothermal gas, containing oxygen and hydrogen sulfide. The aerobic manipulation of the methanogenic microorganisms using cultures of *Methanothermobacter thermautotrophicus* contributes to the efficiency of the method.

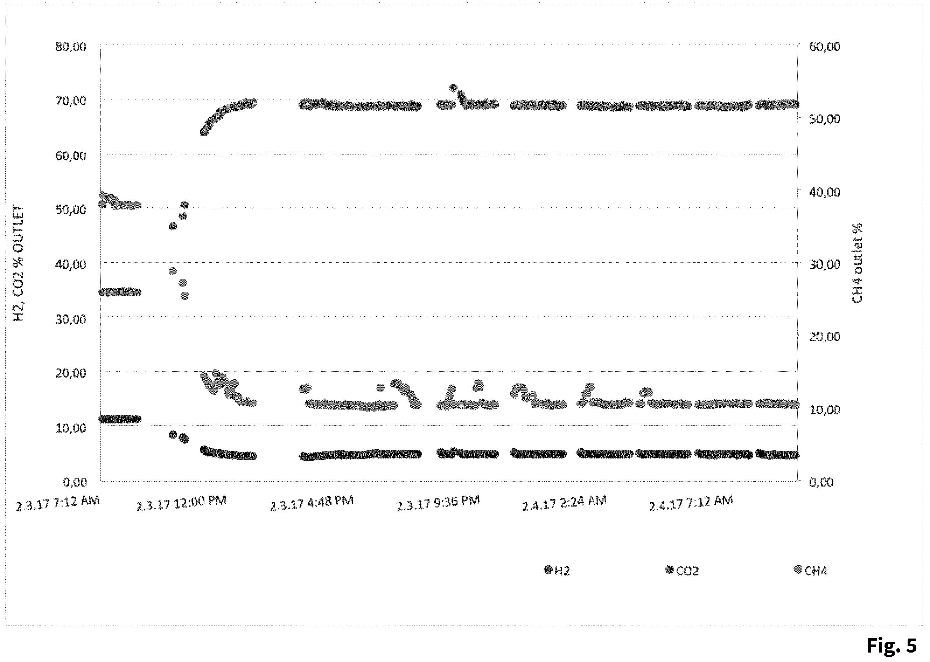

- **Fig. 5:**: Persistence of methanation using only treated geothermal gas, according to example 5 using cultures of *Methanothermobacter thermautotrophicus.* The data show the surprising persistence of methanation even when a very low stoichiometric ratio of H2 to CO₂ was maintained and additional contaminants were added to the culture, according to an embodiment of the method of the present invention.

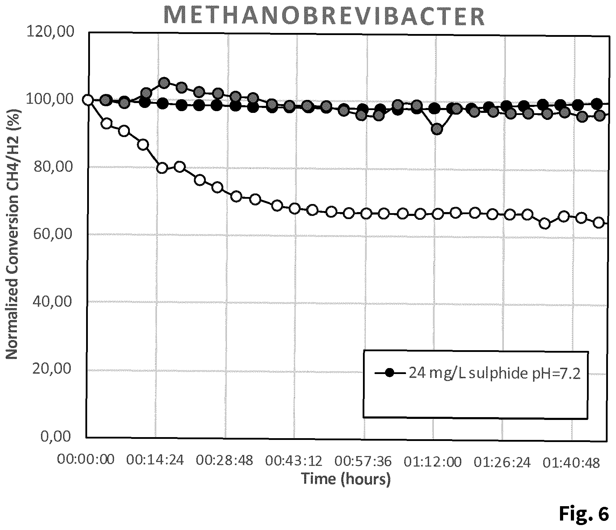

- **Fig. 6:**: Consequences for methanation using *Methanobrevibacter arboriphilus* as biocatalyst following addition of 24 mg/L of S²⁻ (black filled circles), 121 mg/L of S²⁻ (grey filled circles) up to 12.700 mg/L of S²⁻ (unfilled circles). Initial pH values are indicated. Initial methanation was set to 100% and further values were normalized to this value. Each measurement point at a given time of a graph is indicated by a circle and represents the value of one repetition or the average value of two independent repetitions. The data show both the surprising performance in terms of metabolic activity of the culture after the addition of contaminants (Na2S) when the pH regulation is implemented and the clear performance decrease when no pH regulation is implemented.

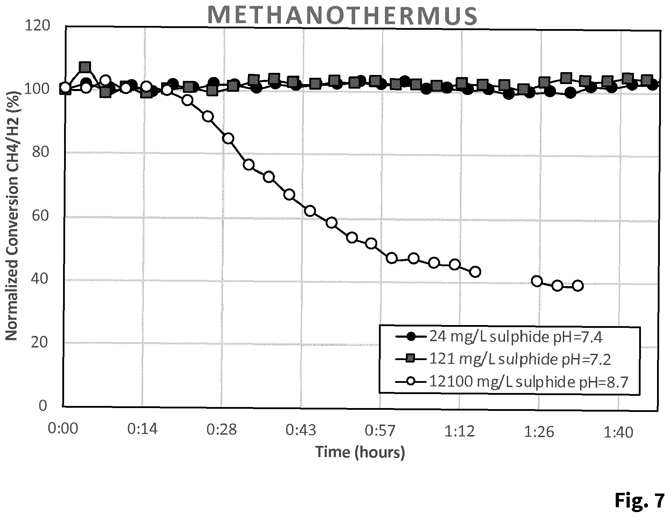

- **Fig. 7:**: Consequences for methanation using *Methanothermus fervidus* as biocatalyst following addition of 24 mg/L of S²⁻ (black filled circles), 121 mg/L of S²⁻ (grey filled circles) up to 12.700 mg/L of S²⁻ (unfilled circles). Initial pH values are indicated. Initial methanation was set to 100% and further values were normalized to this value. Each measurement point at a given time of a graph is indicated by a circle and represents the value of one repetition or the average value of two independent repetitions. The data show both the surprising performance in terms of metabolic activity of the cultures after the addition of contaminants (Na2S) when the pH regulation is implemented and the clear performance decrease when no pH regulation is implemented.

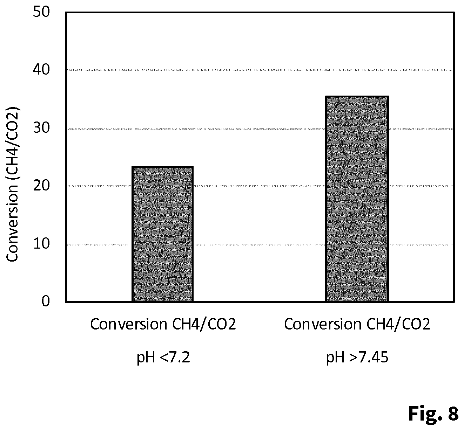

- **Fig. 8:**: Average CO2 conversion rate of a *Methanobrevibacter arboriphilus* culture at a first pH value (pH< 7.2) and after shifting the pH to an alkaline second pH value (pH< 7.45) higher than the first pH value.

The following examples illustrate viable ways of carrying out the described method.

### Example 1: Simulation of hydrogen sulfide effect on standard methanation process using cultures of Methanothermobacter thermautotrophicus

At the beginning of the experiment the biomethanation process using methanogenic archaea (cultures of *Methanothermobacter thermautotrophicus)* was at constant performance conditions (stable biomass concentration of 10 to 12 g/L and volumetric methane productivity of approx. 40 L_{CH4}/L_{reactor}/day). Under such standard conditions the feeding gas input (H2 and CO₂), the biomass of the methanogenic archaea (biocatalysator) as well as pH value (between pH 8.0 and 8.5 - dependent on the amount of dissolved CO₂) were stable.

For the start of the experiment at a defined point in time (see Fig.1 at 189 hours run time) 2000 ppm H2S were continuously added into the hydrogen and carbon dioxide gas supplied. Initially the methane conversion rate was at the expected level when 60 ppm H₂S were added (~90% conversion), but dropped to a value below the detection limit within 192 hours after addition of 2000 ppm H₂S. The culture also turned black upon addition of 2000 ppm H₂S, which indicated the formation of non-dissolvable metal-sulfide complexes, as they are known for the media components nickel, iron and cobalt.

The addition of 2000 ppm H2S lead thus to a sudden and steady decrease in both the biomass concentration and the volumetric methane productivity, of about 50% and 100% respectively. This "collapse" could also not be recovered, by reducing the H2S concentration to only 60 ppm H2S - a value, which is known to be usually tolerated by the process (311 hours run time in Fig.1). Without being bound by the hypothesis, we speculated that the addition of these relatively high amounts of H2S lead to the formation of insoluble sulfide salts with certain metals present in the culture medium. This hypothesis was supported by the black coloring of the biocatalyst suspension upon addition of H2S, it is known, that e.g. iron creates a black, non-soluble salt with sulfide. If these metals are not dissolved but precipitated, they are not available to the biocatalyst and this limitation will cause an effect - as observed - of decrease in biocatalyst biomass concentration and total loss of methanation productivity.

### Example 2: pH regulation enables tolerance of high concentrations of sulfide (S²⁻) up to 24000 mg/L using cultures of Methanothermobacter thermautotrophicus

In Figure 2 a graph reporting the methanation activity of a culture of *Methanothermobacter thermautotrophicus* according to the method of the present invention is shown, as measured in H2 reduction (or conversion). The results of five experiments are reported in the graph, corresponding to the 1-5 in the table and identified by the symbols in the legend, both shown on the right.

In the first 2 experiments, a concentration of 12000 mg/L of sulfide (S²⁻) is administered, in the form of Na2S, to the culture, while providing pH regulation, within the framework described in the method of the present invention. It can be seen in the graph that a high conversion of H2 is achieved in presence of said concentration of 12000 mg/L of sulfide, and such results are steadily maintained for the duration of the experiment.

Experiment 3 shows the results on the conversion of H2 of administering 24000 mg/L of sulfide (S²⁻) to the culture without pH regulation: the pH of the culture drifts to highly alkaline (>12) and the conversion lowers dramatically, followed by a slow rise towards normalization.

Experiments 4 and 5 show the results on the conversion of H2 of administering 24000 mg/L of sulfide (S²⁻) to the culture with pH regulation: the pH of the culture is actively maintained at values within the framework described in the method of the present invention, resulting in an unexpectedly and surprisingly high H2 conversion and steady and continuous performance of the culture in presence of said concentration of 24000 mg/L of sulfide for the duration of the experiment.

From the comparison of the results of experiments 3 with those of experiments 4 and 5 it can be inferred that the culture undergoing pH regulation according to the method of the present invention can not only withstand high concentrations of sulfide without loss of performance or continuity of the methanation process, but also improve on its own performance.

### Example 3: Improving the process by regulating the pH value using cultures of Methanothermobacter thermautotrophicus

To maintain the methanation process performance in the test set up upon addition of H2S, the inventors introduced a further process step of effectively controlling the pH value and thereby insured that during the entire process a pH around 7 was obtained. Under these process conditions using cultures of *Methanothermobacter thermautotrophicus,* the formation of HS⁻ and S²⁻ was found to be much lower and also the formation of the insoluble salts seemed to be reduced.

For this, the process culture - after the experimental set up of Example 1 - was diluted with fresh culture medium by a factor of 1:2 (381 h run time in Fig. 1). In this experiment the dilution approach was used, because it is an easy way to decrease the concentration of unwanted components, such as the previously precipitated metal salts, in the biocatalyst suspension, without having to completely restart the process. Additionally, the pH value was set to a range of 7 (ca. ± 0.3). In this starting phase the H2S supplied was still 60 ppm.

Under the diluted and pH stabilized culture conditions, the biocatalyst culture reinitiated both methane productivity and biomass production (Fig. 1; run time 450h).

At 501 h run time, the H₂S supply was setto 2000 ppm again. With the new pH setting, the negative, previously observed effect of H2S addition on the culture performance and growth could be resolved.

The pH fluctuation between approx. 500 and 630 h is due to the fact, that the pH dosing had to be manually adjusted and the new dosing rate sufficient to maintain pH 7 had to first be determined. Non-sufficient supply of pH-controlling base caused the pH value to drop to the pH values of around pH 6, which also showed in a loss of methane productivity.

### Example 4: Hydrogen sulfide tolerance at concentrations up to 16000 ppm of H2S using cultures of Methanothermobacter thermautotrophicus

With the pH control strategy according to Example 3, also much higher concentrations - e.g. up to 16000 ppm H2S - could be continuously added into the test system and fed together with the hydrogen and carbon dioxide gas supplied to the biomethanation process.

For this, initially the methane conversion rate in cultures of *Methanothermobacter thermautotrophicus* was stabilized at the expected level when 60 ppm H2S were added (-90% conversion) but dropped to a value below the detection limit within 192 hrs after addition of 2000 ppm H2S.

The methanation in the presence of high levels of H₂S was only to be stabilized, when the inventors restarted the process and - manually-decreased the pH value of the culture, which normallywould be between 8 and 8.5, down by at least one log to a pH-value of around 7.

This modification - surprisingly - allowed to run the methanation process with addition of up to 16000 ppm H2S, without any relevant change in the methane productivity.

### Example 5: Hydrogen sulfide tolerance of the methanation process under the influence of additional oxygen using cultures of Methanothermobacter thermautotrophicus

The results according to Example 4 were reproduced in an experimental set up using cultures of *Methanothermobacter thermautotrophicus* where up to 12000 ppm H2S were added. Additionally, O₂ was added in addition to H2S in this experiment, in steps of 1000 ppm/day and up to a final concentration of 5000 and 7000 ppm/day.

The simultaneous addition of H2S and O₂ showed no significant effect on the conversion efficiency (remaining at 90%). This result is the basis and nicely proves the unexpected advantage of the pH control according to the present invention, even when using "real" or at least "treated" geothermal gas in the methanation process.

### Example 6: Methanation process with pretreated geothermal gas using cultures of Methanothermobacter thermautotrophicus

Raw non-condensable fraction of geothermal gas can contain quite high amounts of H2S, in the range of up to 10 times more than the typically stated 30000 ppm H₂S. As in some industrial settings these high amounts are decreased to 30000 ppm by pretreating the raw geothermal gas in the so-called Sulfix process (e.g. at the *Hellisheiði* geothermal power plant, Iceland), in this experimental set the pretreated gas was used for experiments using cultures of *Methanothermobacter thermautotrophicus.* This gas is named "treated geothermal gas".

It is to note that the treated geothermal gas typically contains oxygen (O₂) at concentrations of up to approx. 2% which is a situation that is created by a compressor and would normally not be part of the natural environment of methanogenic archaea.

The long-term effect of such high H2S or O₂ levels, and especially the combination of both, has never been systematically investigated hitherto. However, the tolerance of methanation process according to this invention towards these parameters is a prerequisite to allow usage of the hydrogen (H2) and carbon dioxide (CO₂) present in the non-condensable fraction of treated geothermal gas for methane production.

The typical composition of the treated geothermal gas is shown in Table 1:

**Table 1. Typical composition of treated geothermal gas.**

| | **Gas composition of treated geothermal gas** |
|---|---|
| | Composition (vol%) |
| H₂S | 3.3% |
| CO₂ | 51.5% |
| H₂ | 36.1% |
| N₂ | 6.6% |
| O₂ | 1.1% |
| CH₄ | 1.0% |
| H₂O | 0.3% |
| Sum | 100.0% |

The process was initiated with pure CO₂ and electrolytic H₂ to start the culture before switching to geothermal gas for 3 days. Subsequently, the "pure" CO₂ was replaced by CO₂ from treated geothermal gas and flow rates were adjusted accordingly, in order to maintain the total inlet flows of 0.4 L/min and a stoichiometry of 4.3:1. Consequently, the flow of treated geothermal gas was 0.125 L/min corresponding to a CO₂ flow of approximately 0.065 L/min (53% CO₂ content in the treated geothermal gas). By dilution with additional electrolytic H2, the other gases such as H2S, O₂ and N₂ at the inlet were 0.6 vol.%, 0.4 vol.% and 2.4 vol.%, respectively (Fig. 4). As illustrated in Figure 4, the switch from CO₂ to treated geothermal gas caused a slight drop of the conversion of CO₂ into CH4from 98% to 87%, which was quickly recovered within a few hours and resulted again in a conversion rate above 95%.

### Example 7: Methanation process with treated geothermal gas only using cultures of Methanothermobacter thermautotrophicus

One test should be especially mentioned here, since the experiment, where the addition of electrolytic H2 was completely turned off, was particularly successful.

For this, the process - as described above - was established solely on treated geothermal gas using cultures of *Methanothermobacter thermautotrophicus.* This did allow running the methanation process without an electrolyzer installed on solely (treated) geothermal gas.

The treated geothermal gas had a ratio of H₂:CO₂ of ca. 0.67: 1 thus far below the often believed optimum ratio 4:1. Consequently, incomplete CO₂ conversion was expected. However, the process being adapted with a pH-control did run extremely successfully on the pretreated gas solely.

The "total" CO₂ conversion was around 13.6%, which corresponds to ca. 80% of the theoretical maximum conversion rate (of ca. 16.2%), given this stoichiometry. This very surprising effect demonstrated that the methanation process did operate successfully, even if only treated geothermal gas was used as process feed (Fig. 5).

### Example 8: Methanation at high concentrations of sulfide using cultures of Methanobrevibacter arboriphilus

In Figure 6 three graphs reporting the methanation activity of cultures of *Methanobrevibacter arboriphilus* with and without applying the pH controlling and regulating method of the present invention is shown, as measured in H₂ reduction (or conversion). Three different sulfide concentrations were tested each represents a single graph. Average optical density (OD₆₁₀) of the cultures was 3 to 5.

In a first experiment (black filled circles), a concentration of 24 mg/L of sulfide (S²-) was administered in the form of Na2S, to the culture of the *Methanobacteria* species, while providing pH regulation, within the framework described in the method of the present invention. It can be seen in the corresponding graphs of Fig. 6 that a high methane formation is achieved in presence of said concentration of 24 mg/L of sulfide regulated at an initial pH value of 7.2, and such results are steadily maintained for the duration of the experiment.

Similar results have been observed by using cultures of *Methanobrevibacter arboriphilus* in a second experiment (grey filled circles), when a 5-fold higher concentration of 121 mg/L of sulfide is administered while providing pH regulation at an initial pH value of around 7.2: These conditions resulted in an unexpectedly and surprisingly high methanation and continuous performance of the culture in presence of said concentration of 121 mg/L of sulfide.

In a third experiment the methanation was analyzed when administering 12.700 mg/L of sulfide, respectively, to the culture without any pH regulation. Fig. 6 shows the results at an initial pH value of 8.7, i.e. around pH value 9 (cf. unfilled circles). In this scenario without pH regulation the initial pH increased to a pH value of above 9.0 and consequently the methanation lowers dramatically during the duration of the experiment.

### Example 9: Methanation at high concentrations of sulfide using cultures of Methanothermus fervidus

Comparable as in Figure 6, in Figure 7 three graphs reporting the methanation activity of cultures of *Methanothermus fervidus* with and without applying the pH controlling and regulating method of the present invention is shown, as measured in H2 reduction (or conversion). Three different sulfide concentrations were tested each represents a single graph. Average optical density (OD₆₁₀) of the cultures was 1.5 to 2.5.

The results of the first experiment (black filled circles) given a concentration of 24 mg/L of sulfide (S²-) and of the second experiment (grey filled circles) given a concentration of 121 mg/L of sulfide showed similar results as in the case when using *Methanobrevibacter arboriphilus* as can be seen in Figure 7. I.e., these concentrations of sulfide resulted in a high methane formation, and such results are steadily maintained for the duration of the experiment.

In the third experiment, where the methanation was analyzed when administering 12.700 mg/L of sulfide, respectively, to the culture without any pH regulation similar results as in the case of the third experiment using cultures of *Methanobrevibacter arboriphilus* were received. Fig. 7 shows the results (cf. unfilled circles). In this scenario without pH regulation the initial pH of 8.7 increased to a pH value of above 9.0 and consequently the methanation lowers dramatically during the duration of the experiment.

From the comparison of the results of experiments 1 and 2 with those of experiment 3 of *Methanobrevibacter arboriphilus* and *Methanothermus fervidus* it can be inferred that the cultures of the two further and distinct microorganism genera undergoing pH regulation according to the method of the present invention consistently can withstand high concentrations of sulfide without loss of performance or continuity of the methanation process.

### Example 10: Improved cultivation method

In the attempt to further improve the methanation, the inventors also improved the cultivation techniques of methanogenic microorganisms. In brief, a *Methanobrevibacter arboriphilus* culture was grown at steady conditions for roughly 400 hours at a pH ≤ 7.2 (average 7.1). Under these conditions, an average (CO₂) conversion of 23% was observed.

After this initial starting the cultivation, the pH was increased to be constantly above 7.45 (average 7.6). The surprising effects on methanation over time is depicted in Figure 8. This pH shift to an increased pH resulted in an average (CO₂) conversion of 35% and was thus unexpectedly improved to an average methanation rate advantageously ca. 50% higher than at the respective lower pH condition (cf. Figure 8).

In a similar experiment, where a *Methanobrevibacter arboriphilus* culture was first grown at steady conditions at a pH ≤ 6.5 showed a comparable unexpected and beneficial increase in methanation after the pH was shifted to be constantly above 7.45 (data not shown).

From these experiments the inventors of the present invention concluded - without being bound by theory that - against any prediction of the state of the art knowledge a shift to increased pH values had a beneficial effect on the metabolism and methanation performance of methanogenic microorganisms as shown in the Examples of the present invention for different *Methanobacteria* species.

### References:

Maillacheruvu, K. Y., Parkin, G. F., Peng, C. Y., Kuo, W. C., Oonge, Z. I., Lebduschka, V., Sulfide toxicity in anaerobic systems fed sulfate and various organics, Water Environment Federation, Vol. 65 (2), pp. 100-109 (1993)
Koster, I. W., Rinzema, A., De Vegt, A. L., Letinga, G., Sulfite inhibition of the methanogenic activity of granular sludge at various pH - levels, Water Research, Vol. 20 (12), pp. 1561-1567 (1986)
O'Flaherty, V., Mahony, T., O'Kennedy, R., Colleran, E., Effect of pH on growth kinetics and sulphide toxicity thresholds of a range of methanogenic, syntrophic and sulphate-reducing bacteria, Process Biochemistry, Vol. 33, Issue 5, pp. 555-569 (1998)
Paula Jr., D. R., Foresti, E., Sulfide toxicity kinetics of a uasb reactor, Braz. J. Chem. Eng., Vol. 26 no. 4, pp. 669-675 (2009)
Edgcomb, V. P., Molyneaux, S. J., Saito, M. A., Lloyd, K., Böer, S., Wirsen, C. O., Atkins, M. S., Teske, A., Sulfide Ameliorates Metal Toxicity for Deep-Sea Hydrothermal Vent Archaea, Appl. and Environmental Microbiol., Vol. 70, no. 4, pp. 2551-2555 (2004)
McAnulty, M. J., Poosarla, V.G., Kim, K.-Y., Jasso-Chávez, R., Logan, Br. E., Wood, T. K., Electricity from methane by reversing methanogenesis, Nat. Comm., Vol. 8, art. 15419 (2017)
Procházka, J., Dolejš, P., Máca, J. & Dohányos, M., Stability and inhibition of anaerobic processes caused by insufficiency or excess of ammonia nitrogen, Appl. Microbiol. Biotechnol., Vol. 93, pp. 439-447 (2012).
Anderson I, Djao OD, Misra M, Chertkov O, Nolan M, Lucas S, Lapidus A, Del Rio TG, Tice H, Cheng JF, Tapia R, Han C, Goodwin L, Pitluck S, Liolios K, Ivanova N, Mavromatis K, Mikhailova N, Pati A, Brambilla E, Chen A, Palaniappan K, Land M, Hauser L, Chang YJ, Jeffries CD, Sikorski J, Spring S, Rohde M, Eichinger K, Huber H, Wirth R, Göker M, Detter JC, Woyke T, Bristow J, Eisen JA, Markowitz V, Hugenholtz P, Klenk HP, Kyrpides NC.; Complete genome sequence of Methanothermus fervidus type strain (V24S); Stand Genomic Sci. 2010 Nov 20;3(3):315-24
Stetter KO, Thomm M, Winter J, Wildgruber G, Huber H, Zillig W, Jane-Covic D, König H, Palm P, Wunderl S.. Methanothermus fervidus, sp. nov., a novel extremely thermophilic methanogen isolated from an icelandic hot spring. Zentralbl Bakteriol Parasitenkd Infektionskr HygAbt 1 Orig C2 1981; 2:166-178

## Claims

1. Method to produce methane from CO₂ containing gases in a bioreactor comprising:
a. culturing methanogenic microorganism in a continuous process;
b. providing CO₂ containing gases, comprising H2S and/or O₂;
c. feeding the culture of methanogenic microorganisms with additional H₂ in a stoichiometric ratio of CO₂:H₂ between 1:0.6 to 1:5;
d. controlling and regulating the pH value continuously to be kept at a pH value at a given value of below or at pH 10 by adding suitable amounts of an acid and/or base;
e. collecting methane or a methane enriched gas composition.

2. Method according to claim 1, **characterized in that** the step of culturing the methanogenic microorganism comprises:
i. keepingsaid methanogenic microorganism in a suitable liquid culture medium providing a nitrogen source and salts;
ii. keeping the culture conditions anaerobic or facultatively anaerobic;
iii. optionally stirring the culture;
iv. removing metabolic water from the culture continuously; and
v. keeping the temperatures in a range from 32°C and 90°C or32°C and 85°C at atmospheric pressure.

3. Method according to any of the previous claims 1 to 2, **characterized in that** the methanogenic microorganism is cultured in the presence of additionally added sulfide, preferably in the form of Na2S and/or ammonium, preferably in the form of NH₄OH.

4. Method according to any of the previous claims 1 to 3, **characterized in that** the methanogenic microorganism are cultured up to a density of microorganisms in the culture measured as OD₆₁₀ being at least 14 and up to 60 and corresponding to a dry weight of the microorganisms in the culture of at least 2,5 g/L and up to 20 g/L.

5. Method according to any of the previous claims 1 to 4, **characterized in that** the methanogenic microorganism culture is continuously stabilized or regulated to be kept at a given value of below or at pH 9, of below or at pH 8 or at pH 7 by adding suitable amounts of an acid and/or base;
or that the methanogenic microorganism culture is continuously stabilized or regulated to keep the given pH value by dosing suitable amounts of NaOH or NH₄OH and HCl or H₂SO₄ to the culture.

6. Method according to any of the previous claims 1 to 5, **characterized in that** at least one methanogenic microorganism is selected from the group of Archaea or archaebacteria comprising of *Methanobacterium, Methanobrevibacter, Methanothermobacter, Methanococcus, Methanosarcina, Methanopyrus* or mixtures thereof.

7. Method according to any of the previous claims 1 to 6, **characterized in that** the methanogenic microorganism is anaerobic and/or oxygen tolerant.

8. Method according to any of the previous claims 2 to 7, **characterized in that** in the suitable liquid culture medium of step i. is a moderately saline environment, wherein the concentration of the chloride anion is in the range of 12 mmol/L to 300 mmol/L;
and/or wherein the concentration of NaCl is in the range of 0.4 g/L to 12 g/L, preferably in the range from 3 g/L to 6 g/L and more preferably around 5.6 g/L.

9. Method according to any of the previous claims 1 to 8, **characterized in that** the methanogenic microorganism is selected from the group of naturally selected or natively adapted microorganisms, adapted halophilic microorganisms and genetically engineered microorganisms, all capable of living and thriving in moderately saline environment.

10. Method according to any of the previous claims 1 to 9, **characterized in that** the CO₂ containing gases is the carbon source for the methane production and derives from CO₂ rich emissions and/or waste gas emissions.

11. Method according to any of the previous claims 1 to 10, **characterized in that** the CO₂ containing gas comprises at least 20% CO₂ and/or the CO₂ containing gas comprises up to 5.000 mg/L of H2S and/or the CO₂ containing gas comprises up to, but not more than 5% O₂.

12. Method according to any of the previous claims 1 to 11, **characterized in that** the entire procedure or at least one step is carried out under atmospheric pressure conditions or under pressurized conditions with up to 16 or up to 420 bar.

13. Method according to any of the previous claims, **characterized in that** the collected methane is free of solid contaminants, small solid particles, solid contaminants in suspension, greases, or gaseous contaminants, volatile organic compounds (VOCs) and other trace contaminants.

14. A method to produce methane from CO₂ or CO₂ containing gases in a bioreactor comprising:
a. culturing a methanogenic microorganism with CO₂ and H2 at a first given pH value in a range from pH 5.5 to 7.0 to induce rapid replication of the methanogenic microorganism, followed by
b. continuous culturing said microorganism at a second given pH value in a range from pH 7.1 to 10 to increase methane production compared to the methane production of step a;
c. controlling and regulating the pH value continuously to be at the given value;
d. collecting methane or a methane enriched gas composition.

## Patentansprüche

1. Verfahren zur Herstellung von Methan aus CO₂-haltigen Gasen in einem Bioreaktor, umfassend:
a. Kultivierung eines methanogenen Mikroorganismus in einem kontinuierlichen Prozess;
b. Bereitstellen von CO₂-haltigen Gasen, die H₂S und/oder O₂ umfassen;
c. Füttern der Kulturvon methanogenen Mikroorganismen mit zusätzlichem H₂ in einem stöchiometrischen Verhältnis von CO₂:H₂ zwischen 1:0,6 und 1:5;
d. Steuern und kontinuierliches Regulieren des pH-Wertes, so dass er auf einem pH-Wert mit einem gegebenen Wert von unter oder bei pH 10 gehalten wird, durch die Zugabe geeigneter Mengen einer Säure und/oder Base;
e. Sammeln von Methan oder einer mit Methan angereicherten Gaszusammensetzung.

2. Verfahren nach Anspruch 1, **dadurch gekennzeichnet, dass** der Schritt der Kultivierung des methanogenen Mikroorganismus umfasst:
i. Halten des methanogenen Mikroorganismus in einem geeigneten flüssigen Kulturmedium, das eine Stickstoffquelle und Salze bereitstellt;
ii. Anaerob- oder Fakultativ-Anaerob-Halten der Kulturbedingungen;
iii. optional Rühren der Kultur;
iv. kontinuierliches Entfernen von Stoffwechselwasser aus der Kultur und
v. Halten der Temperaturen in einem Bereich von 32°C bis 90°C oder 32°C bis 85°C bei Atmosphärendruck.

3. Verfahren nach einem der vorhergehenden Ansprüche 1 bis 2, **dadurch gekennzeichnet, dass** der methanogene Mikroorganismus in Gegenwart von zusätzlich zugesetztem Sulfid, vorzugsweise in Form von Na₂S und/oder Ammonium, vorzugsweise in Form von NH₄OH, kultiviert wird.

4. Verfahren nach einem der vorhergehenden Ansprüche 1 bis 3, **dadurch gekennzeichnet, dass** der methanogene Mikroorganismus in der Kultur bis zu einer Dichte von Mikroorganismen in der Kultur, gemessen als OD₆₁₀, von mindestens 14 und bis zu 60 kultiviert wird, die einem Trockengewicht der Mikroorganismen in der Kultur von mindestens 2,5 g/L und bis zu 20 g/L entspricht.

5. Verfahren nach einem der vorhergehenden Ansprüche 1 bis 4, **dadurch gekennzeichnet, dass** die methanogene Mikroorganismenkultur durch die Zugabe geeigneter Mengen einer Säure und/oder Base kontinuierlich stabilisiert und/oder reguliert wird, um auf einem gegebenen Wert von unter oder bei pH 9, von unter oder bei pH 8 oder bei pH 7 gehalten zu werden,
oder dass die methanogene Mikroorganismenkultur durch Dosieren geeigneter Mengen von NaOH oder NH₄OH und HCl oder H₂SO₄ in die Kultur kontinuierlich stabilisiert oder reguliert wird, um den gegebenen pH-Wert einzuhalten.

6. Verfahren nach einem der vorhergehenden Ansprüche 1 bis 5, **dadurch gekennzeichnet, dass** mindestens ein methanogener Mikroorganismus ausgewählt ist aus der Gruppe der Archaea oder Archaebakterien, umfassend *Methanobacterium, Methanobrevibacter, Methanothermobacter, Methanococcus, Methanosarcina, Methanopyrus* oder Mischungen davon.

7. Verfahren nach einem der vorhergehenden Ansprüche 1 bis 6, **dadurch gekennzeichnet, dass** der methanogene Mikroorganismus anaerob und/oder sauerstoffverträglich ist.

8. Verfahren nach einem der vorhergehenden Ansprüche 2 bis 7, **dadurch gekennzeichnet, dass** in dem geeigneten flüssigen Kulturmedium aus Schritt i. eine mäßigsalzhaltige Umgebung vorliegt, wobei die Konzentration des Chloridanions im Bereich von 12 mmol/L bis 300 mmol/L liegt;
und/oder wobei die Konzentration von NaCl im Bereich von 0,4 g/L bis 12 g/L, vorzugsweise im Bereich von 3 g/L bis 6g/L und stärker bevorzugt um 5,6 g/L liegt.

9. Verfahren nach einem der vorhergehenden Ansprüche 1 bis 8, **dadurch gekennzeichnet, dass** der methanogene Mikroorganismus ausgewählt ist aus der Gruppe der natürlich ausgewählten oder nativ geeigneten Mikroorganismen, der angepassten halophilen Mikroorganismen und der gentechnisch veränderten Mikroorganismen, die alle in einer mäßig salzhaltigen Umgebung leben und gedeihen können.

10. Verfahren nach einem der vorhergehenden Ansprüche 1 bis 9, **dadurch gekennzeichnet, dass** die CO₂-haltigen Gase die Kohlenstoffquelle für die Methanproduktion sind und aus CO₂-reichen Emissionen und/oder Abgasemissionen stammen.

11. Verfahren nach einem der vorhergehenden Ansprüche 1 bis 10, **dadurch gekennzeichnet, dass** das CO₂-haltige Gas mindestens 20% CO₂ umfasst und/oder das CO₂-haltige Gas bis zu 5.000 mg/L H₂S umfasst und/oder das CO₂-haltige Gas bis zu, jedoch nicht mehr als 5% O₂ umfasst.

12. Verfahren nach einem der vorhergehenden Ansprüche 1 bis 11, **dadurch gekennzeichnet, dass** das gesamte Verfahren oder mindestens ein Schritt unter Atmosphärendruckbedingungen und/oder unter Druckbedingungen mit bis zu 16 oder bis zu 420 bar durchgeführt wird.

13. Verfahren nach einem der vorhergehenden Ansprüche, **dadurch gekennzeichnet, dass** das gesammelte Methan frei von festen Verunreinigungen, kleinen Feststoffpartikeln, festen Verunreinigungen in Suspension, Fetten oder gasförmigen Verunreinigungen, flüchtigen organischen Verbindungen (VOCs) und anderen Spurenverunreinigungen ist.

14. Verfahren zur Herstellung von Methan aus CO₂ oder CO₂-haltigen Gasen in einem Bioreaktor, umfassend:
a. Kultivierung eines methanogenen Mikroorganismus mit CO₂ und H₂ bei einem ersten gegebenen pH-Wert in einem Bereich von pH 5,5 bis 7,0, um eine schnelle Replikation des methanogenen Mikroorganismus zu induzieren, gefolgt von
b. kontinuierlicher Kultivierung des Mikroorganismus bei einem zweiten gegebenen pH-Wert in einem Bereich von pH 7,1 bis 10, um die Methanproduktion gegenüber der Methanproduktion aus Schritt a zu erhöhen;
c. Steuern und kontinuierliches Regulieren des pH-Wertes auf den gegebenen Wert;
d. Sammeln von Methan oder einer mit Methan angereicherten Gaszusammensetzung.

## Revendications

1. Méthode destinée à produire du méthane à partir de gaz contenant du CO₂ dans un bioréacteur comprenant :
a. la culture d'un microorganisme méthanogène dans un processus continu ;
b. la livraison de gaz contenant du CO₂, comprenant de l'H₂S et/ou de l'O₂ ;
c. l'alimentation de la culture de microorganismes méthanogènes avec de l'H₂ supplémentaire dans un rapport stœchiométrique de CO₂:H₂ entre 1:0,6 et 1:5 ;
d. le contrôle et la régulation de la valeur de pH en continu afin de la conserver à une valeur de pH à une valeur donnée inférieure ou égale au pH 10 en additionnant des volumes convenables d'un acide et/ou d'une base ;
e. la collecte de méthane ou d'une composition gazeuse enrichie au méthane.

2. Méthode selon la revendication 1, **caractérisée en ce que** l'étape de culture du microorganisme méthanogène comprend :
i. la conservation dudit microorganisme méthanogène dans un milieu de culture liquide convenable présentant une source d'azote et des sels ;
ii. la conservation des conditions de culture anaérobies ou facultativement anaérobies ;
iii. en option, l'agitation de la culture ;
iv. le retrait en continu de l'eau métabolique de la culture ; et
v. la conservation des températures dans une plage de 32 °C à 90 °C ou de 32 °C à 85 °C à une pression atmosphérique.

3. Méthode selon une quelconque des revendications précédentes 1 à 2, **caractérisée en ce que** le microorganisme méthanogène est cultivé en présence de sulfure additionné en supplément, de préférence sous la forme de Na₂S et/ou d'ammonium, de préférence sous la forme de NH₄OH.

4. Méthode selon une quelconque des revendications précédentes 1 à 3, **caractérisée en ce que** les microorganismes méthanogènes sont cultivés jusqu'à une densité de microorganismes dans la culture mesurée comme OD₆₁₀,étant d'au moins 14 et allant jusqu'à 60 et correspondant à un poids à sec des microorganismes dans la culture d'au moins 2,5 g/L et de jusqu'à 20 g/L.

5. Méthode selon une quelconque des revendications précédentes 1 à 4, **caractérisée en ce que** la culture de microorganismes méthanogènes est stabilisée en continu ou régulée afin d'être conservée à une valeur donnée inférieure ou égale au pH 9, ou inférieure ou égale au pH 8 ou au pH 7 en additionnant des volumes convenables d'un acide et/ou d'une base ;
ou **en ce que** la culture de microorganismes méthanogènes est stabilisée en continu ou régulée afin d'être conservée à la valeur de pH donnée en dosant des volumes convenables de NaOH ou de NH₄OH et d'HCI ou d'H₂SO₄ dans la culture.

6. Méthode selon une quelconque des revendications précédentes 1 à 5, **caractérisée en ce qu'**au moins un microorganisme méthanogène est sélectionné à partir du groupe d'Archaea ou d'archaebacteria comprenant *Methanobacterium, Methanobrevibacter, Methanothermobacter, Methonococcus, Methanosarcina, Methanopyrus* ou des mélanges de ceux-ci.

7. Méthode selon une quelconque des revendications précédentes 1 à 6, **caractérisée en ce que** le microorganisme méthanogène est anaérobie et/ou tolérant l'oxygène.

8. Méthode selon une quelconque des revendications précédentes 2 à 7, **caractérisée en ce que** le milieu de culture liquide convenable de l'étape i. est un environnement modérément salin, dans lequel la concentration de l'anion de chlorure est dans la plage de 12 mmol/L à 300 mmol/L ; et/ou dans lequel la concentration de NaCl est dans la plage de 0,4 g/L à 12 g/L, de préférence dans la plage de 3 g/L à 6 g/L et plus de préférence autour de 5,6 g/L.

9. Méthode selon une quelconque des revendications précédentes 1 à 8, **caractérisée en ce que** le microorganisme méthanogène est sélectionné à partir du groupe de microorganismes sélectionnés naturellement ou adaptés originairement, de microorganismes halophiles adaptés et de microorganismes modifiés génétiquement, tous capables de vivre et de prospérer dans un environnement modérément salin.

10. Méthode selon une quelconque des revendications précédentes 1 à 9, **caractérisée en ce que** les gaz contenant du CO₂ sont la source de carbone pour la production de méthane et proviennent d'émissions riches en CO₂ et/ou d'émissions de gaz rejetés.

11. Méthode selon une quelconque des revendications précédentes 1 à 10, **caractérisée en ce que** le gaz contenant du CO₂ comprend au moins 20 % de CO₂ et/ou le gaz contenant du CO₂ comprend jusqu'à 5.000 mg/L d'H₂S et/ou le gaz contenant du CO₂ comprend jusqu'à, mais pas plus de 5 % d'O₂.

12. Méthode selon une quelconque des revendications précédentes 1 à 11, **caractérisée en ce que** la procédure entière ou au moins une étape est réalisée dans des conditions de pression atmosphérique ou dans des conditions pressurisées avec jusqu'à 16 ou jusqu'à 420 bar.

13. Méthode selon une quelconque des revendications précédentes, **caractérisée en ce que** le méthane collecté est exempt de contaminants solides, de petites particules solides, de contaminants solides en suspension, de graisses ou de contaminants gazeux, de composés organiques volatiles (COV) et d'autres contaminants à l'état de traces.

14. Méthode destinée à produire du méthane à partir de CO₂ ou de gaz contenant du CO₂ dans un bioréacteur comprenant :
a. la culture d'un microorganisme méthanogène avec du CO₂ et de l'H₂ à une première valeur de pH donnée dans une plage de pH 5,5 à 7,0 afin d'induire une réplication rapide du microorganisme méthanogène, suivie par
b. la culture continue dudit microorganisme à une seconde valeur de pH donnée dans une plage de pH 7,1 à 10 afin d'augmenter la production de méthane comparée à la production de méthane de l'étape a ;
c. le contrôle et une régulation de la valeur pH en continu afin qu'elle soit à la valeur donnée ;
d. la collecte de méthane ou d'une composition gazeuse enrichie au méthane.
